(19) Europäisches Patentamt
European Patent Office
Office européen des brevets

(11) EP 4 431 513 A1

(12) EUROPEAN PATENT APPLICATION
published in accordance with Art. 153(4) EPC

(43) Date of publication:
**18.09.2024 Bulletin 2024/38**

(21) Application number: **22891956.9**

(22) Date of filing: **08.11.2022**

(51) International Patent Classification (IPC):
***C07D 498/20*** *(2006.01)* ***A61K 31/537*** *(2006.01)*
***A61P 31/18*** *(2006.01)*

(86) International application number:
**PCT/CN2022/130550**

(87) International publication number:
**WO 2023/083162 (19.05.2023 Gazette 2023/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.11.2021 CN 202111336306**
**03.11.2022 CN 202211367919**

(71) Applicants:
- **Nanjing Aidea Pharmaceutical Technology Co., Ltd.**
  **Nanjing, Jiangsu 210044 (CN)**
- **Jiangsu Aidea Pharmaceutical Co., Ltd.**
  **Yangzhou, Jiangsu 225123 (CN)**

(72) Inventors:
- **SHI, Yian**
  **Yangzhou, Jiangsu 225123 (CN)**
- **TIAN, Zongyong**
  **Yangzhou, Jiangsu 225123 (CN)**
- **CHEN, Lulu**
  **Yangzhou, Jiangsu 225123 (CN)**
- **HU, Tianjin**
  **Yangzhou, Jiangsu 225123 (CN)**
- **FU, Heliang**
  **Yangzhou, Jiangsu 225123 (CN)**

(74) Representative: **Cabinet Laurent & Charras**
**Le Contemporain**
**50 Chemin de la Bruyère**
**69574 Dardilly Cedex (FR)**

# (54) POLYCYCLIC CARBAMOYL PYRIDONE DERIVATIVE, PREPARATION METHOD THEREFOR, AND PHARMACEUTICAL COMPOSITION THEREOF

(57) The present invention relates to the technical field of drug for treatment of AIDS. Disclosed are a polycyclic carbamoyl pyridone derivative, a preparation method therefor, and pharmaceutical composition thereof. The derivative comprises comprises the compound represented by formula (I) -1 or formula (I) -2, or an isomer, pharmaceutically acceptable salt, hydrate or solvate thereof. At least one group of functional groups in (1) - (3) below and a ring A form a spirolcycle or spiroheterocycle: (1) $R_1$ and $R_2$; (2) $R_3$ and $R_4$; (3) $R_5$ and $R_6$; the polycyclic carbamoyl pyridone derivative can selectively inhibit the activity of HIV integrase, and thus can be used for preventing and treating AIDS.

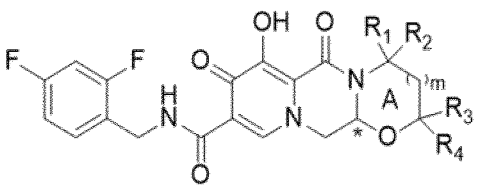

(I) -1

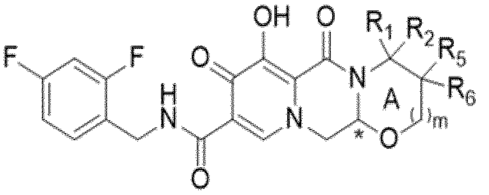

(I) -2

## Description

### TECHNICAL FIELD

The present invention relates to the technical field of drugs for treatment of AIDS, and specifically, the present invention relates to a polycyclic carbamoyl pyridone derivative, a preparation method therefor and a pharmaceutical composition thereof.

### BACKGROUND OF THE INVENTION

AIDS, the full name of "acquired immunodeficiency syndrome" (AIDS), is a malignant infectious disease with high mortality rate caused by human immunodeficiency virus (HIV) infection. HIV virus can specifically attack and destroy human immune cells, resulting the damage of the human immune system, the gradual loss of related immune functions, and then gradually becoming the target of many diseases, leading to a variety of serious opportunistic infections, tumors, and eventually developping into AIDS.

HIV is a retrovirus whose genetic information is stored in ribonucleic acid (RNA). HIV selectively recognizes and invades immune cells with CD4 molecules on their surface(CD4+lymphocytes), and enters host cells through interaction of their surface proteins with CD4 molecules. Once HIV enters the host cell, single stranded RNA, the genetic material of HIV, is used as a template to form complementary double stranded DNA under the action of reverse transcriptase,which enters the host cell nucleus andis integrated into the host cell genome after being catalyzed by integrase, and is subsequently transcribed, translated, and cleaved into new viral proteins under the action of protease, ultimately new mature viral particles are formed and released outside the cell, infecting more host cells,and the cycle repeats itself.

The absence of no functional analogues of integrase in human cells and the relatively low toxicityof drug during use, making integrase become an ideal target for anti HIV drugs. Integrase inhibitors produce antiviral effects mainly by inhibiting the HIV integrase, which is required for virus replication, and preventing covalent insertion or integration of the HIV genome into the host cell genome in the early stages of infection. The integrase inhibitors have significant therapeutic effect, they can reduce the number of viruses quickly, and generally have good tolerance. They have been increasingly supplemented as first-line treatment options by relevant guidelines at home and abroad. Currently, the launched integrase inhibitors include the early first-generation of Elvitegravir (EVG) and Raltegravir (RAL), the recently launched second-generation Dolutegravir (DTG), the compound Biktatvy containing Bitegravir(BIC), and the prolonged action preparations of Cabotegravir (CAB).

Early launched integrase inhibitors have a lower resistance barrier, and one or two mutations can reduce virus sensitivity and exhibit high cross-resistance. Subsequent products such as Dolutegravir have a high resistance barrier and have been widely used since launch. Although integrase inhibitors have a high resistance barrier, resistance is inevitable like protease inhibitors and reverse transcriptase inhibitors.

With current medical technology, life long medication is required once infected with HIV. Lifetime treatment has become a long-term goal for highly effective antiretroviral therapy (HARRT), but there are still many issues to be considered, and drug resistance is an issue that cannot be ignored, which is a key factor affecting the success of HARRT.

Therefore, it is necessary to develop novel integrase inhibitors to increase oral bioavailability, improve clinical toxic side effects, enhance drug resistance barriers, and provide patients with better drug choice and better compliance, which is of great significance.

On this basis, the present invention is proposed.

### SUMMARY OF THE INVENTION

The purpose of the present invention is to provide a polycyclic carbamoyl pyridone derivative, a preparation method therefor, and a pharmaceutical composition thereof. The polycyclic carbamoyl pyridone derivative can selectively inhibit the activity of HIV integrase, thus can be used to prevent and treat AIDS.

The present invention is realized in this way:

In the first aspect, the present invention provided is a polycyclic carbamoyl pyridone derivative, comprising a compound shown in formula (I) -1 or formula (I) -2, an isomer, a pharmaceutically acceptable salt, a hydrate, or a solvate thereof,

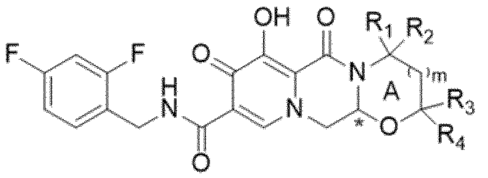

Formula (I) -1;

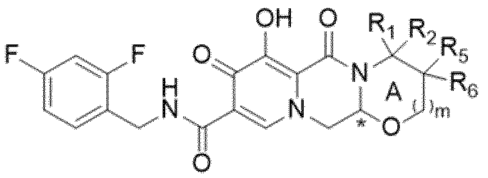

Formula (I) -2,

wherein $R_1$, $R_2$, $R_3$, $R_4$, $R_5$and $R_6$ are each independently selected from the group consisting of hydrogen, halogen, substituted or unsubstituted alkyl, substituted or unsubstituted cycloalkyl, substituted or unsubstituted alkenyl, substituted or unsubstituted alkynyl, $-OR_7$, substituted or unsubstituted aryl, substituted or unsubstituted monocyclic heteroaryl, substituted or unsubstituted monocyclic heterocyclyl, and cyano;
and at least one group of functional groups in (1) - (3) below together with the carbon atoms to which ring A connected forms a spirolcycle or spiroheterocycle:
(1) $R_1$ and $R_2$; (2) $R_3$ and $R_4$; (3) $R_5$ and $R_6$; that is to say, when the polycyclic carbamoyl pyridone derivative is a compound shown in formula (I) -1, the functional group formed by $R_1$ and $R_2$ or the functional group formed by $R_3$ and $R_4$ together with the carbon atom to which ring A connected forms a spirolcycle or a spiroheterocycle; when the polycyclic carbamoyl pyridone derivative is a compound shown in formula (I) -2, the functional group formed by $R_1$ and $R_2$ or the functional group formed by $R_5$ and $R_6$ together with the carbon atom which ring A connected form a spirolcycle or a spiroheterocycle.

$R_7$ is selected from the group consisting of hydrogen, substituted or unsubstituted alkyl, substituted or unsubstituted alkenyl, and substituted or unsubstituted alkynyl;
m is any natural number between 0 and 5. When m is 0, it indicates that A ring is a 5-membered ring.

In the second aspect, the present invention provided is a method for preparing the polycyclic carbamoyl pyridone derivative as described in any one of the aforementioned embodiments, and the polycyclic carbamoyl pyridone derivative is synthesized according to the following synthesis route:

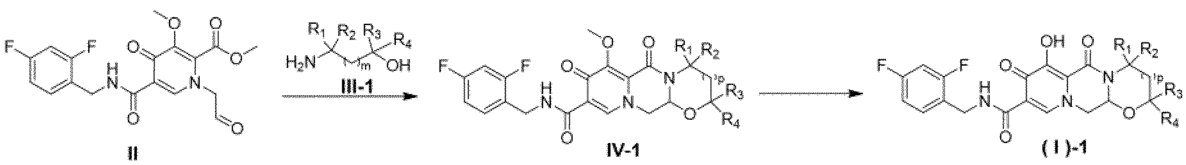

II III-1 IV-1 ( I )-1

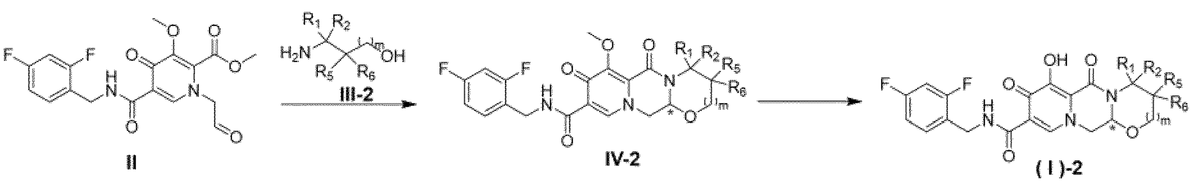

II III-2 IV-2 ( I )-2

In the third aspect, the present invention provided is a pharmaceutical composition comprising a polycyclic carbamoyl pyridine derivative as described in any of the aforementioned embodiments, an isomer, a pharmaceutically acceptable salt, a hydrate, or a solvate thereof, and pharmaceutically acceptable carriers.

The present invention has the following beneficial effects:

1. The polycyclic carbamoyl pyridone derivative of the present invention can selectively inhibit the activity of HIV integrase, and then can be widely used to prevent and treat AIDS;
2. It has excellent permeability and PK properties, which are favorable to become medication, it also can reduce

the frequency of administration and increase patient's compliance with medication;

3. It has low cytotoxicity, which improves safety of medication and reduces the risk of adverse reactions.

## DETAILED DESCRIPTION OF THE INVENTION

In order to clarify the object, technical solution, and advantages of the embodiments of the present invention, a clear and complete description of the technical solution in the embodiments of the present invention will be provided. If specific conditions are not described in embodiments, they shall be performed under conventional conditions or conditions recommended by the manufacturer. The reagents or instruments used without specific manufacturer are conventional products that can be commercially available.

An embodimentof the present invention provides a polycyclic carbamoyl pyridone derivative, comprising a compound shown in formula (I) -1 or formula (I) -2, or an isomer, a pharmaceutically acceptable sals, a hydrate, a solvate thereof,

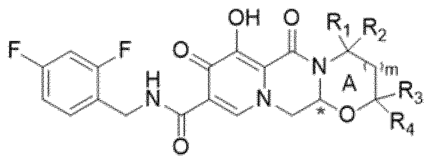

Formula (I) -1;

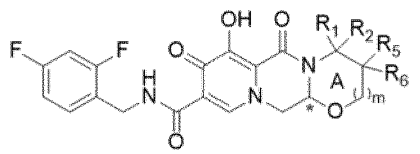

Formula (I) -2,

wherein $R_1$, $R_2$, $R_3$, $R_4$, $R_5$ and $R_6$ are each independently selected from the group consisting ofhydrogen, halogen, substituted or unsubstituted alkyl, substituted or unsubstituted cycloalkyl, substituted or unsubstituted alkenyl, substituted or unsubstituted alkynyl, $-OR_7$, substituted or unsubstituted aryl, substituted or unsubstituted monocyclic heteroaryl, substituted or unsubstituted monocyclic heterocyclyl, and cyano; and at least one group of functional groups in (1) - (3) below together with the carbon atom to which ring A connected forms a spirolcycle or spiroheterocycle: (1) $R_1$ and $R_2$; (2) $R_3$and $R_4$; (3) $R_5$ and $R_6$; $R_7$ is selected from the group consisting of hydrogen, substituted or unsubstituted alkyl, substituted or unsubstituted alkenyl, and substituted or unsubstituted alkynyl; m is any natural number between 0 and 5.

Further, $R_1$, $R_2$, $R_3$, $R_4$, $R_5$ and $R_6$ are each independently selected from any one of the group consisting of hydrogen, halogen, C1-4 alkyl, halogenated C1-C4 alkyl, C2-C6 alkenyl, C2-C6 alkynyl, $-OR_5$, C3-C8 cycloalkyl, C6-C10 aryl, 5-6-membered monocyclic heteroaryl, 4-6 membered saturated monocyclic heterocyclyl, and cyano; and at least one group of functional groups in (1) - (3) together with the carbon atom to which ring A connected forms a 3-8-membered spirolcycle or a 3-8-membered spiroheterocycle: (1) $R_1$ and $R_2$; (2) $R_3$ and $R_4$; (3) $R_5$ and $R_6$; $R_7$ is selected from any one of the group consisting of hydrogen, C1-4alkyl, C2-C6 alkenyl, C2-C6 alkynyl, or halogenated C1-C4 alkyl; m is 0, 1, 2, or 3.

Further, the tertiary carbon atom to which ring A and the fused pyrazine ring connected in the compounds shown in Formula (I) -1 and (I) -2 is chiral; the configuration of the compounds shown in Formula (I) -1 and (I) -2 can be a single configuration or a mixture of multiple configurations. And isomers include at least one of tautomers, cis-trans isomers, mesoisomers and optical isomers with enantiomeric or non enantiomeric relationships. Specifically, the tertiary carbon atom to which A ring and the fused pyrazine ring connected has an S configuration.

Further, $R_1$ and $R_2$ are selected from the group consisting of C1-4alkyl and C3-C5 cycloalkyl, specifically methyl or cyclopropyl, more specifically the methyl with R configuration; $R_3$ and $R_4$ are selected from the group consisting of hydrogen, C3-C6 cycloalkyl and C3-C6 heterocyclyl specifically hydrogen, cyclopropyl, cyclopentyl, cyclohexyl, and 6-membered heterocyclyl comprising oxygen, $R_5$ and $R_6$ are selected from the group consisting of hydrogen, C3-C6 cycloalkyl and C3-C6 heterocyclyl, specifically hydrogen, cyclopropyl, cyclobutyl, cyclopentyl, cyclohexyl and 6-membered heterocyclyl comprising oxygen.

Further, the polycyclic carbamoyl pyridone derivative is selected from any one of the compounds shown in the following structural formulas:

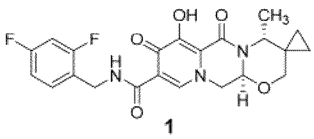
1
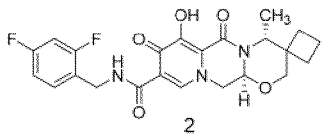
2
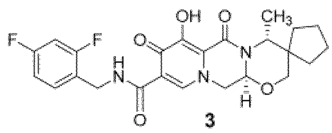
3
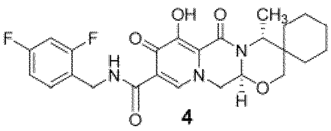
4
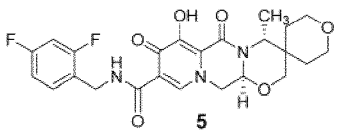
5
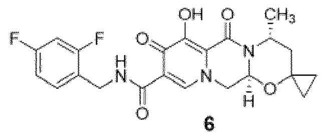
6
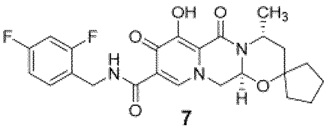
7
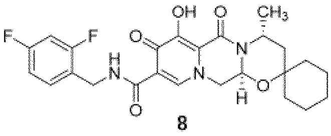
8
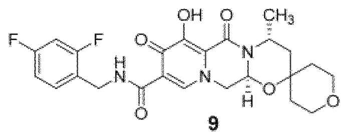
9
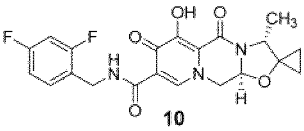
10
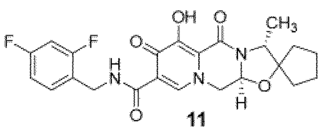
11
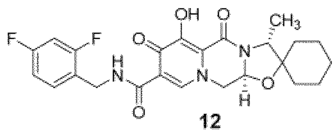
12
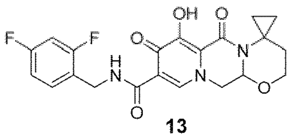
13
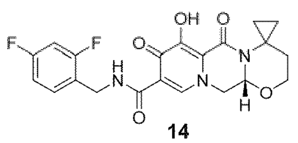
14
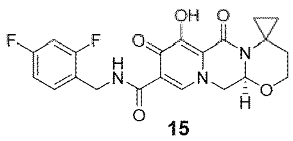
15
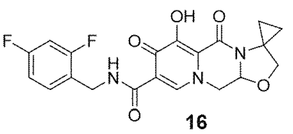
16
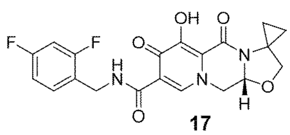
17
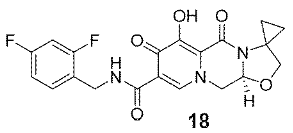
18

,

Preferably is

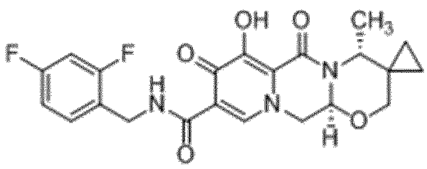

.

The terms mentioned in the embodiments of the present invention have the following definitions:
"Halogen" refers to fluorine, chlorine, bromine or iodine.

"C1-C4 alkyl" refers to methyl, ethyl, propyl, isopropyl, butyl, isobutyl, sec-butyl or tert butyl.

"Cycloalkyl" refers to amonovalent group derived from a monocyclic or polycyclic, saturated or partially unsaturated aliphatic carbon cyclic compound, C3-C8 cycloalkyl include, but are not limited to cyclopropyl, cyclobutyl, cyclopentyl, cyclohexyl or cyclohexyl,cyclooctyl, cyclooctenyl, cyclopropenyl, cyclobutenyl, cyclopentenyl, cyclohexenyl or cyclohexenyl.

"Alkenyl" refers to a monovalent group derived from a hydrocarbon group. C2-C6 alkenyl refers to an alkenyl group having 2 to 6 carbon atoms and at least one carbon-carbon double bond, including but not limited to vinyl, propenyl, butenyl, 2-methyl-2-butene, 2-methyl-2-pentene, and the like.

"Alkynyl" refers to an alkynyl group having 2 to 6 carbon atoms and at least one carbon-carbon triple bond, including but not limited to ethynyl, propinyl, 1- butynyl, 2-butynyl, and the like.

"Aryl" refers to an aromatic cyclic hydrocarbon group having one or more aromatic, fused or non-fused carbon ring system, such as phenyl and naphthyl. "Aryl" can be substituted or unsubstituted, and when substituted, the substituent is preferably one or more of the following groups, independently selected from the group consisting of alkyl, alkenyl, alkynyl, alkoxy, alkylamino, halogen, thiol, hydroxyl, nitro,cyano, and the like.

"Heteroaryl" refers to a 5-6-membered monocyclic heteroaryl group, or a bicyclic heteroaryl fused with a benzene, pyridine or pyrrole, having 1 to 4 heteroatoms selected from the group consisting of N, S and O. It can be partially saturated, such as pyridyl, pyrimidyl, furyl, and the like.

"Spirolcycle" refers to a polycyclic group that shares a carbon atom between single rings, which can have one or more double bonds, but none of the rings have a complete conjugated $\pi$ - electron system. Based on the number of rings, spirolcycles are categorized as bicyclic spirolcycle or polycyclic spirolcycle, preferably bicyclic spirolcycle.

"Spiroheterocycle" refers to a polycyclic group that shares a carbon atom between single rings, wherein one or two ring atoms are selected from the heteroatoms consisting of $NR_6$ ($R_6$ is hydrogen, oxygen, alkyl or halogenated alkyl), oxygen or S(O)n (n is 0, 1 or 2), and the remaining ring atoms are carbon. These can contain one or more double bonds, but none of the rings have a complete conjugated $\pi$ - electron system. Based on the number of rings, spiroheterocycles are categorized as bicyclic spiroheterocycles or polycyclic spiroheterocycle, preferably bicyclic spirolcycle.

"The pharmaceutically acceptable salt" refers to the relatively non-toxic acid addition salt or base addition salt of the compound provided in the embodiments of the present invention. The acid addition salt is a salt formed by the compound shown in formula (I) -1 or (I) -2 provided in the embodiments of the present invention and a suitable inorganic or organic acid. For example, hydrochloride, fumarate, etc.

The base addition salt is a salt formed by the compound shown in formula (I) -1 or (I) -2 provided in the embodiments of the present invention and a suitable inorganic or organic base. For example, sodium salts, methylamine salts, etc.

In the second aspect, the embodiments of the present invention provide a preparation method for the above-mentioned polycyclic carbamoyl pyridone derivatives, and the polycyclic carbamoyl pyridone derivatives are synthesized according to the following synthesis route:

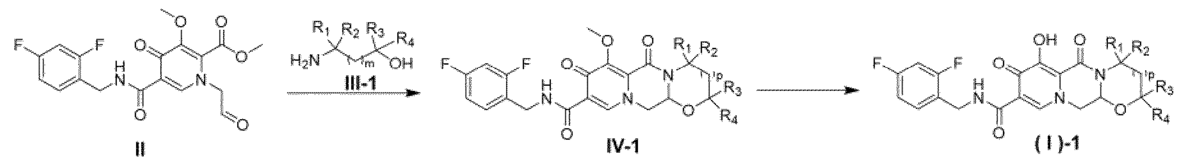

II III-1 IV-1 ( I )-1

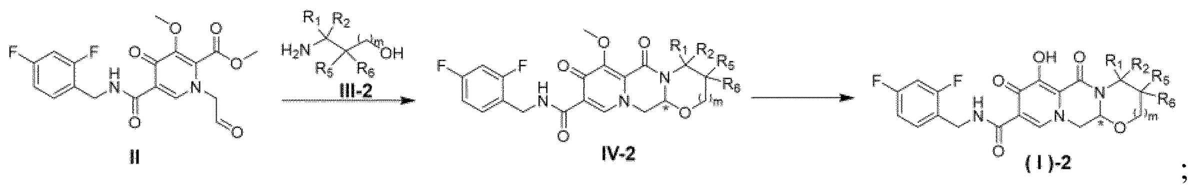

II III-2 IV-2 ( I )-2 ;

The preparation process of the compound shown in formula (I) -1 or (I) -2 provided in the embodiments of the present invention is as follows: using compound II as the raw material, subjecting to intramolecular cyclization with compound III-1 or III-2 in the present of an acid catalysis to obtain intermediate IV-1 or IV-2, then the intermediate IV-1 or IV-2 undergo a demethylation reaction.

Wherein, the compound shown in formula (II) can be prepared by the the method described in Organic Letter, 2015, 17564-567, as well as the other conventional methods in this field.

The acid catalysts used in the preparation of intermediate IV-1 or IV2 are organic acid catalysts or inorganic acid catalysts, such as formic acid, acetic acid, trifluoroacetic acid, trifluoromethanesulfonic acid, p-toluenesulfonic acid, magnesium sulfate, sodium sulfate, 4A molecular sieve or magnesium trifluoromethanesulfonate, etc., preferaly acetic acid.

Specifically, the conditions for preparing intermediate IV-1 include: the molar ratio between compound II and compound III-1 is 1:1-1:5, preferably 1:1.5-1:3; the molar ratio between compound II and acid catalyst is 1:1-1:10, preferably 1:4-1:8; the temperature is 30-100 °C, preferably 60-80 °C; the reaction solvent is acetonitrile, toluene, etc., preferalyacetonitrile; the time is 5 minutes -16 hours;

The conditions for preparing intermediate IV-2 include: the molar ratio betweencompound II and compound III-2 is 1:1-1:5, preferably 1:1.5 to 1:3; the molar ratio betweencompound II and acid catalyst is 1:1-1:10, preferably 1:4-1:8; the temperature is 30-100 °C; preferably 60-80 °C; the reaction solvent is acetonitrile, toluene, etc., preferably acetonitrile; the time is 5 minutes -16 hours.

The demethylating reagents used in the preparation of the compounds shown in formula (I) -1 or (I) -2 are: boron tribromide, sodium bromide, sodium iodide, magnesium bromide, lithium bromide, magnesium chloride, preferably lithium bromide.

Specifically, the conditions for preparing the compound shown in formula (I) -1 include: the molar ratio between intermediate IV-1 and demethylating reagent is 1:1-1:10, preferably 1:4-1:6; the reaction temperature is 30-100 °C, preferably 50-70 °C; the reaction solvents are dichloromethane, tetrahydrofuran, etc., preferably tetrahydrofuran; the reaction time is several minutes to several hours, preferably 5 minutes to 16 hours.

The conditions for preparing the compound shown in formula (I) -2 include: the molar ratio between intermediate IV-2 and demethylating reagent is 1:1-1:10, preferably 1:4-1:6; the reaction temperature is 30-1:100 °C, preferably 50-70 °C; the reaction solvents are dichloromethane, tetrahydrofuran, etc., preferably tetrahydrofuran; the reaction time is several minutes to several hours, preferably 5 minutes to 16 hours.

In the third aspect, the present invention provides a pharmaceutical composition comprising a polycyclic carbamoyl pyridone derivative as described in any one of the aforementioned embodiments, an isomer, a pharmaceutically acceptable salt, a hydrate, or a solvate thereof, and pharmaceutically acceptable carriers. Wherein, the pharmaceutically acceptable carriers are selected from the group consisting of injection water, freeze-dried powder excipients and oral preparation excipients.

The features and performance of the present invention will be further detailed described in conjunction with the following examples.

Example 1

An example of the present invention provides a polycyclic carbamoyl pyridone derivative having the structural formula as follow:

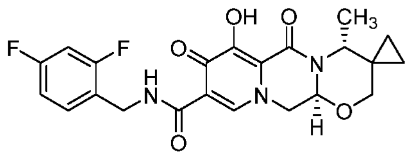

;

named: (4R,12aS)-N-(2,4-difluorophenyl)-7-hydroxy-4-methyl-6,8-dioxo-6,8,12,12a-tetrahydro-2H,4 H spiro[cyclopropane-1,3-pyridino[1,2:4,5]pyrazine[2,1-b][1,3]oxazine] -9'-formamide.

The present example provides a preparation method for the above-mentioned polycyclic carbamoyl pyridone derivative, comprising:

Preparation of intermediate **1a**:

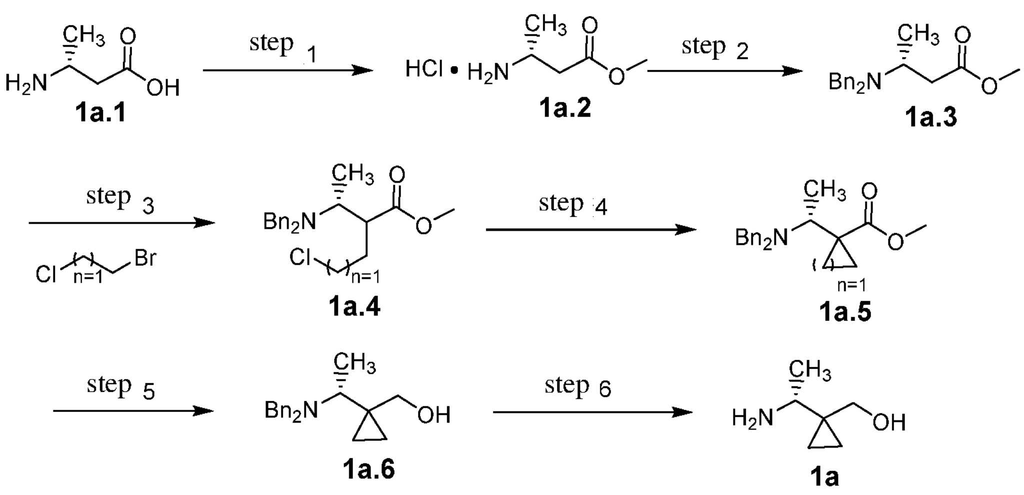


**1a.1** **1a.2** **1a.3** **1a.4** **1a.5** **1a.6** **1a**

;

Step 1: Dichlorosulfoxide (20.4 g, 172 mmol) was added to 160 mL methanol, and cooled down to 0 °C.

Compound **1a.1** (12 g, 116 mmol) was added dropwise to the reaction solution, and stirred at room temperature for 5 hours after addition. After the reaction was completed, it was concentrated under reduced pressure to remove solvent and obtained a concentrate, then the concentrate was added with methanol/dichloromethane (1:1), and concentrated consecutively and multiply times to remove residual dichlorosulfoxide to obtain an off white solid **1a.2** (14.8 g).

$^1$H NMR (400 MHz, CDC13) δ 8.34 (br s, 3H), 3.79 (br s, 1H), 3.69 (s, 3H), 2.95 (d, J=15.9 Hz, 1H), 2.72 (d, J=15.8 Hz, 1H), 1.46 (s, 3H); 13C NMR (100 MHz, CDC13) δ 171.0, 52.4, 45.1, 38.2, 18.7.

Step 2: Compound **1a.2** (10 g, 65 mmol) and sodium bicarbonate (35.8 g, 426.8 mmol) were added to a mixed solvent of 120 mL tetrahydrofuran and 80 mL DMSO, finally benzyl bromide (29.2 g, 170.7 mmol) was added, and the reaction was stirred at 90-100 °C for 12 hours. After cooled down to room temperature, the reaction was quenched with water and ethyl acetate, separated to obtain an organic layer, washed with saturated sodium chloride solution, dried over anhydrous sodium sulfate to obtain a mixture. The mixture was purified by silica gel column chromatography and eluted with ethyl acetate and petroleum ether (1:19) to obtain a white solid **1a.3** (19 g, 98%).

$^1$H NMR (400 MHz, CDC13) δ 7.40-7.28 (m, 8H), 7.27-7.21 (m, 2H), 3.69 (d, J=13.7 Hz, 2H), 3.63 (s, 3H), 3.47 (d, J=13.7 Hz, 2H), 3.40-3.28 (m, 1H), 2.68 (dd, J=14.0, 8.1 Hz, 1H), 2.32 (dd, J=14.0, 6.9 Hz, 1H), 1.13 (d, J=6.7 Hz, 3H) 13C NMR (100 MHz, CDC13) δ 172.8, 140.1, 128.9, 128.2, 126.9, 53.4, 51.5, 50.9, 39.2, 14.0..

Step 3: Compound **1a.3** (0.5 g, 1.7 mmol) was added to 4 mL anhydrous THF, protected by nitrogen gas, and cooled down to -78 °C. 2.6 mL LDA (2M THF solution, 3.0 equiv.) was added dropwise, and stirred for 1-2 hours. Terminally substituted bromochloroalkane was added dropwise at -78 °C, and n=1 was selected for compound in Example 1, i.e 1-bromo-2-chloroethane (1.45g, 10.1 mmol, 6 equiv.). After the addition was completed, the reaction was transferred to room temperature and stirred for 24 hours. It was then quenched with ammonium chloride solution, extracted with ethyl acetate, washed with saturated sodium chloride solution, dried overanhydrous sodium sulfate, and concentrated under reduced pressure to obtain a mixture. The mixture was purified by silica gel column chromatography, eluted with ethyl acetate and petroleum ether (1:19) to obtain a oily substance **1a.4** (0.9 g), which was directly used for the next reaction.

Step 4: Compound **1a.4** (0.8 g) was added to5 mL anhydrous THF and DMPU (1:1), and cooled down to -78 °C under a nitrogen atmosphere. 5.6 mL LiHMDS (1M THF solution, 2.5 equiv.) was added dropwise, and the reaction was stirred at room temperature for 4-5 hoursafter addition.It was then quenched with ammonium chloride solution, extracted with ethyl acetate, washed with saturated sodium chloride solution, driedoveranhydrous sodium sulfate, and concentrated under reduced pressure to obtain a mixture. The mixure was purified by aluminum trioxide column chromatography, eluted with ethyl acetate and petroleum ether (1:19) to obtain a colorless oily substance **1a.5** (0.43 g, two-step yield 30%).

$^1$H NMR (400 MHz, CDC13) δ 7.32-7.16 (m, 10H), 3.85 (d, J=13.6 Hz, 2H), 3.55 (s, 3H), 3.40 (d, J=13.6 Hz, 2H), 3.37 (q, J=6.9 Hz, 1H), 1.30-1.21 (m, 1H), 0.96 (d, J=6.9 Hz, 3H), 0.89-0.76 (m, 2H), 0.63-0.54 (m, 1H).

Step 5: Compound **1a.5** (0.42 g) was added to 10 mL anhydrous ether, and cooled down to 0 °C under a nitrogen atmosphere. Lithium aluminum tetrahydrogen (75 mg, 1.96 mmol, 1.5 equiv.) was added, and the reaction was stirred for 2-3 hours. After the reaction was completed, it was slowly quenched with water at 0 °C, extracted with ethyl acetate, washed with saturatedsodium chloride, dried over anhydrous sodium sulfate, concentrated under reduced pressure, purified by alumina column chromatography,eluted with ethyl acetate and petroleum ether (1:9) to obtain a colorless oily substance **1a.6** (0.25 g, 65%).

$^1$H NMR (400 MHz, CDC13) δ 7.44-7.20 (m, 10H), 4.01 (d, J=13.0 Hz, 2H), 3.52 (dd, J=11.2, 1.7 Hz, 1H), 3.37 (q, J=6.8 Hz, 1H), 3.30 (d, J=13.0 Hz, 2H), 3.19 (d, J=11.2 Hz, 1H), 0.80 (d, J=6.8 Hz, 3H), 0.82-0.72 (m, 1H), 0.69-0.61 (m, 1H), 0.33-0.23 (m, 1H), 0.13-0.05 (m), 1H); 13C NMR (100 MHz, CDC13) δ 139.0, 129.4, 128.7, 127.4, 72.3, 55.5, 54.1, 22.9, 7.0, 6.5, 5.9.

Step 6: Compound **1a.6** (0.25 g, 0.84 mmol) was added to 5 mL methanol, then 80 mg $Pd(OH)_2/C$ (50% hydrate with 20% loaded carbon)was added, and the reaction was stirred at room temperature under a hydrogen atmosphere (balloon) for 12 hours. After the reaction was completed, the carbon was removed by filtrating with diatomaceous earth, and the filtrate was concentrated under reduced pressure to obtain a colorless oily compound **1a** (90 mg, 93%).

$^1$H NMR (400 MHz, CDC13) δ 4.18 (dd, J=11.2, 1.8 Hz, 1H), 3.48 (s, 1H), 2.94 (dd, J=11.3, 1.3 Hz, 1H), 2.61 (qd, J=6.6, 1.2 Hz, 1H), 1.23 (d, J=6.6 Hz, 3H), 0.69-0.60 (m, 1H), 0.51-0.42 (m, 1H), 0.33-0.20 (m, 2H); 13C NMR (100 MHz, CDC13) δ 68.4, 54.8, 25.7, 19.9, 11.4, 7.6.

Preparation of intermediate **1b**:

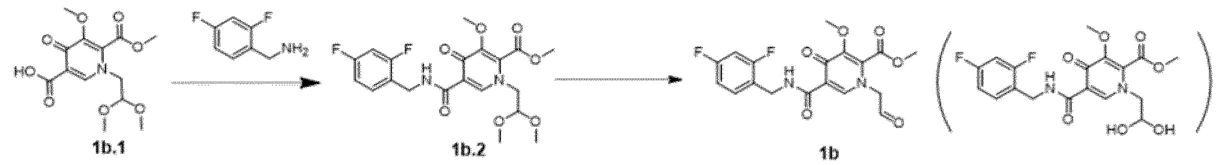

1b.1 1b.2 1b

Main product

;

Step 1: Compound **1b.1** (50 g, 158.6 mmol) and N,N'-carbonyldiimidazole (30.8 g, 190.3 mmol) were added to 500 mL acetonitrile, protected by nitrogen gas, and the reaction was carried out for 2-3 hours under stirred at a temperature rised to reflux. It was then cooled downed to 0-10 °C, and a mixed solution of 2,4-difluorobenzylamine (27.2 g, 190.3 mmol) and 30 mL acetonitrile were added slowly. After the addition was completed,the reaction was carried out at room temperature for 1 hour. After the reaction was completed, it was quenched with 200 mL water, evaporated by vacuum rotary evaporation to remove the acetonitrile, diluted with 300 mL ethyl acetate, washed with 300 mL 2M hydrochloric acid as well assaturated sodium bicarbonate solution twice, respectively, dried over anhydrous sodium sulfate, and concentrated under reduced pressure to obtain a pale yellow solid, which was further washed with ether and dried to obtain an off white solid **1b.2** (53.4 g, 77%).

$^1$H NMR (400 MHz, CDC13) δ 10.39 (t, J=5.4 Hz, 1H), 8.42 (s, 1H), 7.41-7.35 (m, 1H), 6.85-6.77 (m, 2H), 4.62 (d, J=5.6 Hz, 2H), 4.50 (t, J=4.8 Hz, 1H), 4.04 (d, J=4.8 Hz, 2H), 3.99 (s, 3H), 3.96 (s, 3H), 3.39 (s, 6H).

Step 2: Compound **1b.2** (20 g, 45.4 mmol) was added to 100 mL formic acid, and stirred at 60 °C for 3 hours. After the reaction was completed, the solvent was removed by rotary evaporation, it was thenthen diluted with acetonitrile, concentrated several times, added with 80 mL methyl tert butyl ether, stirred for 10-12 hours for crystallization, filtered, and dried to obtain a white solid **1b** (17.2 g).

$^1$H NMR (400 MHz, DMSO-d6) (hydrate) δ 10.31 (t, J=5.8 Hz, 1H), 8.46 (s, 1H), 7.45-7.39 (m, 1H), 6.28-6.72 (m, 1H), 7.10-7.05 (m, 1H), 6.39 (d, J=5.8 Hz, 2H), 4.96 (t, J=5.6 Hz, 1H), 4.53 (d, J=5.7 Hz, 2H), 3.95 (d, J=5.1 Hz, 2H), 3.93 (s, 3H), 3.81 (s, 3H).

Compound **1b** is an acetal deprotected product ($^1$H NMR shows that hydrate is the main form), and no matter which structure does not affect the ring closing reaction, and compound **1b** (MW: 412.1) was calculated based on hydrate in the following example.

Preparation of compound **1**

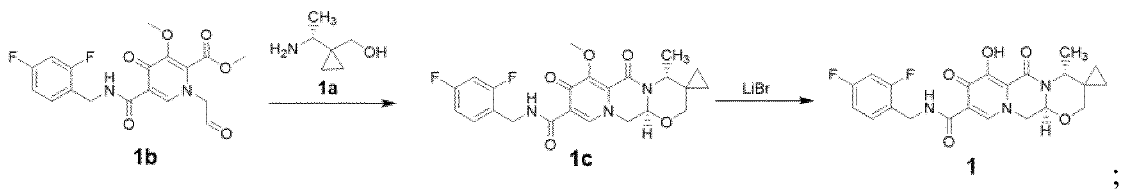


1b 1c 1

;

Step 1: Compound **1b** (160 mg, 0.39 mmol), Compound**1a** (90 mg, 0.78 mmol) and acetic acid (140 mg, 2.34 mmol) were added to 2 mL acetonitrile, and stirred at 70-80 °C for 5-6 hours. After the reaction was completed, it was then washed with sodium bicarbonate aqueous solution, extracted with ethyl acetate, dried over anhydrous sodium sulfate, and concentrated under reduced pressure to obtain intermediate **1c,** which was directly used for the next reaction. MS m/z (ESI): 460.2 [M+1]$^+$.

Step 2: Intermediate **1c** was added to anhydrous THF, LiBr (4.0 equiv.) was added, and stirred at 70-80 °C for 5-6 hours. After the reaction was completed, it was then quenched with 0.5M sulfuric acid solution, extracted with ethyl acetate, washed with sodium bicarbonate solution, dried over anhydrous sodium sulfate, and concentrated to obtain a mixture. The mixuture was purified by silica gel column chromatography, and eluted gradiently with dichloromethane/methanol to obtain Compound **1** (an off white solid, 128 mg, two-step yield 74.0%). MS m/z (ESI): 446.2 [M+1]$^+$.

$^1$H NMR (400 MHz, DMSO-d6) δ 12.51 (br s, 1H), 10.36 (t, J=5.9 Hz, 1H), 8.53 (s, 1H), 7.42-7.36 (m, 1H), 7.27-7.21 (m, 1H), 7.09-7.04 (m, 1H), 5.53 (t, J=4.6 Hz, 1H), 4.59 (dd, J=14.1, 3.9 Hz, 1H), 4.55 (d, J=5.9 Hz, 2H), 4.49 (dd, J=14.0, 5.0 Hz, 1H), 4.33 (d, J=11.8 Hz, 1H) 4.09 (q, J=6.9 Hz, 1H), 3.10 (d, J=11.8 Hz, 1H), 1.37 (d, J=7.0 Hz, 3H), 0.56-0.52 (m, 3H), 0.44-0.42 (m, 1H).

Example 2

An example of the present invention provides a polycyclic carbamoyl pyridone derivative having the structural formula as follows:

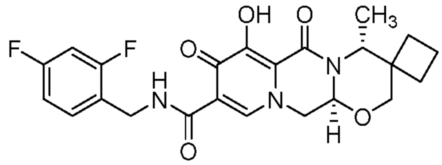

;

named:(4'R,12a'S)-N-(2,4-difluorophenyl)-7'-hydroxy-4'-methyl-6',8'-dioxo-6',8',12',12a'-tetr ahydro-2'H,4'H-spiro[cyclobutane-1,3'-pyridino[1',2':4,5]pyrazine[2,1-b][1,3]pyrazine]-9'-for mamide.

The present example provides a preparation method for the above-mentioned polycyclic carbamoyl pyridone derivative, comprising:

Preparation of intermediate **2a**

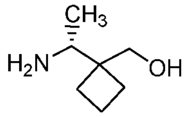

( **2a** ):

referring to the synthesis of intermediate **1a** in Example 1, wherein, the bromochloroalkane was selected as 1-bromo-3-chloropropane with n=2 in step 3, and then subjected to LDA catalyzed substitution, and ring closing reaction under LiHMDS sequentially to obtain cyclobutane spiro intermediate, followed by reduction and deprotection reaction, and then intermediate **2a** (colorless oil, 490mg) was obtained.

$^1$H NMR (400 MHz, CDC13) δ 3.89 (dd, J=11.1, 1.2 Hz, 1H), 3.67 (dd, J=11.0, 1.2 Hz, 1H), 3.43 (s, 1H), 3.29 (qd, J=6.6, 1.2 Hz, 1H), 2.15-2.00 (m, 1H), 1.97-1.75 (m, 3H), 1.71-1.60 (m, 1H), 1.52-1.41 (m, 1H), 1.01 (d, J=6.6 Hz, 3H); 13C NMR (100 MHz, CDCl3) δ 67.9, 55.5, 44.4, 27.9, 26.3, 16.8, 14.9.

Synthesis of compound **2:**

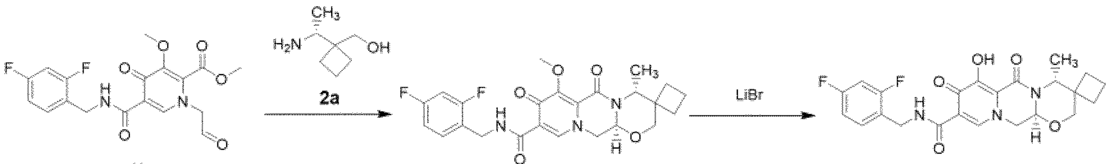


**1b** **2c** **2**

;

Step 1: Compound **1b** (412 mg, 1 mmol), compound **2a** (260 mg, 2 mmol)and acetic acid (360 mg, 6 mmol) were added to 4 mL acetonitrile, and stirred at 70-80 °C for 6-7 hours. After the reaction was completed, it was washed with sodium bicarbonate aqueous solution, extracted with ethyl acetate, dried over anhydrous sodium sulfate, and concentrated under reduced pressure to obtain intermediate **2c** which was directly used for the next reaction. MS m/z (ESI): 474.2 $[M+1]^+$.

Step 2: Intermediate **2c** was added to anhydrous THF, LiBr (4.0 equiv.) was added, and stirred at 70-80 °C for 7-8 hours. After the reaction was completed, it was quenched with 0.5M sulfuric acid solution, extracted with ethyl acetate, washed with sodium bicarbonate solution, dried over anhydrous sodium sulfate, and concentrated to obtain a mixture. The mixture was purified by silica gel column chromatography, eluted gradiently with dichloromethane/methanol to obtain Compound **2** (an off white solid, 260 mg, two-step yield 56.6%). MS m/z (ESI): 460.2 $[M+1]^+$.

$^1$H NMR (400 MHz, CDC13) δ 12.48 (br s, 1H), 10.38 (t, J=5.84 Hz, 1H), 8.32 (s, 1H), 7.39-7.33 (m, 1H), 6.84-6.77 (m, 2H), 5.18 (dd, J=6.0, 4.1 Hz, 1H), 4.88 (q, J=7.0 Hz, 1H), 4.65 (dd, J=15.9, 6.1 Hz, 1H), 4.61 (dd, J=15.9, 6.0 Hz, 1H), 4.28 (dd, J=13.6, 4.1 Hz, 1H), 4.12 (dd, J=13.5, 5.9 Hz, 1H), 3.92 (d, J=12.2 Hz, 1H), 3.87 (d, J=11.8 Hz,

1H), 2.07-1.55 (m, 6H), 1.23 (d, J=7.0 Hz, 3H).

Example 3

An example of the present invention provides a polycyclic carbamoyl pyridone derivative having the structural formulaas follows:

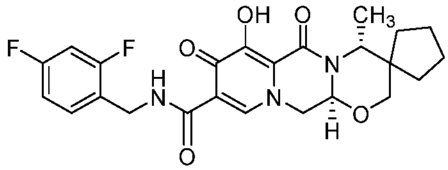
;

named: (4'R,12a'S)-N-(2,4-difluorophenyl)-7'-hydroxy-4'-methyl-6',8'-dioxo-6',8',12',12a'-tetrahydro-2'H,4'H-spiro[cyclopentane-1,3'-pyridino[1',2':4,5]pyrazine[2,1-b][1,3]oxazine]-9' -formamide.

The present example provides a preparation method for the above-mentioned polycyclic carbamoyl pyridone derivative, comprising:

Preparation of intermediate **3a**

( 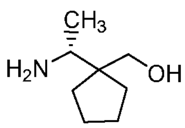
**3a** ):

referring to the synthesis of intermediate **1a** in Example 1, wherein, the bromochloroalkane was selected as 1-bromo-4-chlorobutane with n=4 in step 3, and then subjected to LDA catalyzed substitution, and ring closing reaction under LiHMDS sequentially to obtain cyclopentane spiro intermediate, followed by reduction and deprotection reaction, and then intermediate **3a** (colorless oil, 320mg) was obtained.

$^1$H NMR (400 MHz, CDC13) δ 3.73 (dd, J=10.9, 1.3 Hz, 1H), 3.45 (s, 1H), 3.34 (dd, J=10.9, 1.3 Hz, 1H), 3.06 (qd, J=6.6, 1.3 Hz, 1H), 2.01-1.89 (m, 1H), 1.72-1.50 (m, 4H), 1.40-1.20 (m, 3H), 1.18 (d, J=6.6 Hz, 3H); 13C NMR (100 MHz, CDC13) δ 68.1, 56.6, 49.5, 33.7, 32.1, 25.3, 25.2, 19.0.

Synthesis of compound **3:**

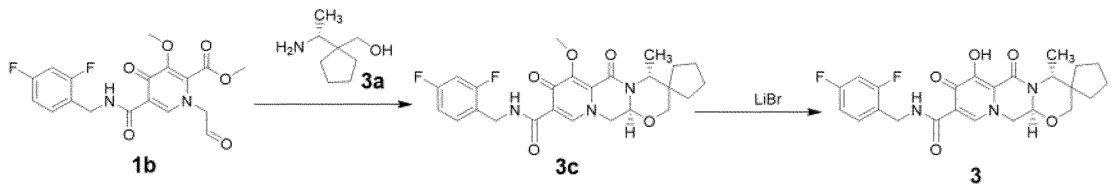

;

Step 1: Compound **1b** (412 mg, 1 mmol), compound **3a** (311 mg, 2.17 mmol) and acetic acid (360 mg, 6 mmol) were added to 4 mL acetonitrile, and stirred at 70-80 °C for 6-7 hours. After the reaction was completed, it was then washed with sodium bicarbonate aqueous solution, extracted with ethyl acetate, dried over anhydrous sodium sulfate, and concentrated under reduced pressure to obtainintermediate **3c,** which was directly used for the next reaction. MS m/z (ESI): 488.2 $[M+1]^+$.

Step 2: Intermediate **3c** was added to anhydrous THF, LiBr (5.0 equiv.) was added, and stirred at 70-80 °Cfor 8 hours. LiBr (5.0 equiv.) was supplementary added again, and the reaction was carried out for 12 hours. After the reaction was completed, it was then quenched with 0.5M sulfuric acid solution, extracted with ethyl acetate, washed with sodium bicarbonate solution, dried with anhydrous sodium sulfate, and concentrated to obtain a mixture. The mixture was purified by silica gel column chromatography, and eluted gradiently with dichloromethane/methanol to obtain Compound **2** (faint yellow solid, 170 mg, two-step yield 36.0%). MS m/z (ESI): 474.2 $[M+1]^+$.

$^1$H NMR (400 MHz, CDC13) δ 12.48 (br s, 1H), 10.40 (t, J=5.8 Hz, 1H), 8.33 (s, 1H), 7.39-7.33 (m, 1H), 6.84-6.77 (m, 2H), 5.24 (dd, J=6.1, 4.2Hz, 1H), 4.69-4.59 (m, 3H), 4.28 (dd, J=13.6, 4.2 Hz, 1H), 4.13 (dd, J=13.5, 6.2 Hz, 1H), 3.82 (d, J=11.2 Hz, 1H), 3.56 (d, J=11.6 Hz), 1H), 1.87-1.16 (m, 8H), 1.33 (d, J=7.04 Hz, 3H).

Example 4

Anexample of the present invention provides a polycyclic carbamoyl pyridone derivative having the structural formula as follows:

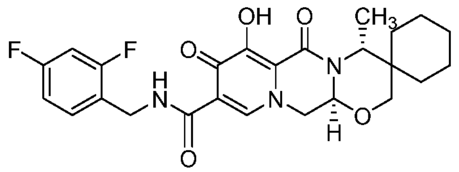

;

named: (4'R,12a'S)-N-(2,4-difluorophenyl)-7'-hydroxy-4'-methyl-6',8'-dioxo-6',8',12',12a'-tetrahydro-2'H,4'H-spiro[cyclohexane-1,3'-pyridino[1',2':4,5]pyrazine[2,1-b][1,3]oxazine]-9'-formamide.

The present example provides a preparation method for the above-mentioned polycyclic carbamoyl pyridone derivative, comprising:

Preparation of intermediate **4a**

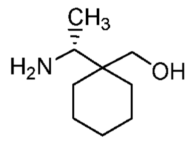

( **4a** ):

referring to the synthesis of intermediate **1a** in Example 1, wherein, the bromochloroalkanewas selected as 1-bromo-5-chloropentane with n=5 in step 3, and then subjected to LDA catalyzed substitution, and ring closing reaction under LiHMDS sequentially to obtain cyclohexane spiro intermediate, followed by reduction and deprotection reaction, and thenintermediate **4a** (colorless oil, 130mg) was obtained.

$^1$H NMR (400 MHz, CDC13) δ 3.77 (d, J=11.1 Hz, 1H), 3.59 (dd, J=11.2, 1.4 Hz, 1H), 3.03 (qd, J=6.6, 1.4 Hz, 1H), 1.93-1.80 (m, 1H), 1.63-1.39 (m, 4H), 1.35-1.25 (m, 4H), 1.14 (d, J=6.6 Hz, 3H), 1.08-0.97 (m, 1H); 13C NMR (100 MHz, CDC13) δ 66.5, 55.6, 38.5, 30.9, 30.4, 26.4, 21.7, 21.5, 17.4.

Synthesis of compound **4:**

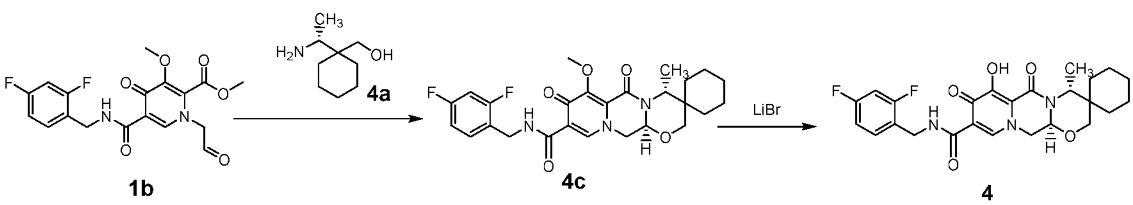


**4a**

**1b** **4c** **4** ;

Step 1: Compound **1b** (173 mg, 0.42 mmol), compound **4a** (130 mg, 0.84 mmol) and acetic acid (185 mg, 2.5 mmol) were added to 2 mL acetonitrile, and stirred at 70-80 °C for 7-8 hours. After the reaction was completed, it was then washed with sodium bicarbonate aqueous solution, extracted with ethyl acetate, dried over anhydrous sodium sulfate, and concentrated under reduced pressure to obtain intermediate **4c,** which was directly used for the next reaction. MS m/z (ESI): 502.2 [M+1]+.

Step 2: Intermediate **4c** was added to anhydrous THF, LiBr (4.0 equiv.) was added, and stirred at 70-80 °C for 22 hours. After the reaction was completed, it was then quenched with 0.5M sulfuric acid solution, extracted with ethyl acetate, washed with sodium bicarbonate solution, dried over anhydrous sodium sulfate, and concentrated to obtain a

mixture. The mixture was purified by silica gel column chromatography, and eluted gradiently with dichloromethane/methanol to obtain Compound **4** (pale yellow solid, 90 mg, two-step yield 43.9%). MS m/z (ESI): 488.2 $[M+1]^+$.

$^1H$ NMR (400 MHz, CDC13) δ 12.52 (br s, 1H), 10.41 (t, J=8.96 Hz, 1H), 8.36 (s, 1H), 7.38-7.35 (m, 1H), 6.84-6.77 (m, 2H), 5.17 (t, J=4.8 Hz, 1H), 4.87 (q, J=7.0 Hz, 1H), 4.68-4.58 (m, 2H), 4.32 (dd, J=13.6, 3.8 Hz, 1H), 4.15 (dd, J=13.6, 5.4 Hz, 1H), 3.73 (d, J=12.0 Hz, 1H) H), 3.67 (d, J=12.0 Hz, 1H), 1.57-1.08 (m, 10H), 1.31 (d, J=7.08 Hz, 3H).

Example 5

An example of the present invention provides a polycyclic carbamoyl pyridone derivative having the structural formula was as follows:

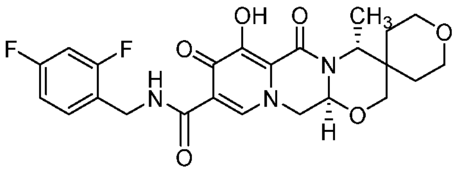

;

named: (4'R,12a'S)-N-(2,4-difluorophenyl)-7'-hydroxy-4'-methyl-6',8'-dioxo-2,3,5,6,6',8',12', 12a'-octahydro-2'H,4'H-spiro[pyran-4,3'-pyridino[1',2':4,5]pyrazine[2,1-b][1,3]oxazine]-9'-fo rmamide.

The present example provides a preparation method for the above-mentioned polycyclic carbamoyl pyridone derivative, comprising:

Preparation of intermediate **5a**

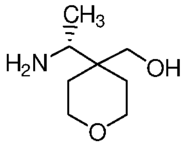

( **5a** ):

referring to the synthesis of intermediate **1a** in Example 1, wherein, the bromochloroalkanes used in step 3 was 1-bromo-2- (2-chloroethoxy) ethane, and then subjected to LDA catalyzed substitution, and ring closing reaction under LiHMDS sequentially to obtain pyranospiro intermediate, followed by reduction and deprotection reaction, and thenintermediate **4a** (colorless oil, 0.29g) was obtained.

$^1H$ NMR (400 MHz, CDC13) δ 3.80-3.75 (m, 3H), 3.74-3.68 (m, 1H), 3.64 (td, J=11.8, 2.6 Hz, 1H), 3.43 (td, J=11.8, 3.0 Hz, 2H), 2.95 (q, J=6.6 Hz, 1H), 2.00-1.92 (m, 1H), 1.53-1.41 (m, 1H), 1.38-1.28 (m, 1H), 1.23-1.55 (m, 1H), 1.12 (d, J=6.6 Hz, 3H); 13C NMR (100 MHz, CDC13) δ 64.1, 63.68, 63.65, 57.1, 36.6, 30.5, 17.0.

Synthesis of compound **5:**

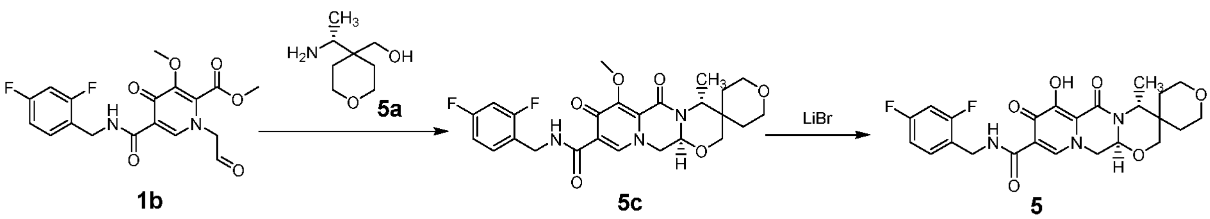


1b 5c 5

Step 1: Compound **1b** (412 mg, 1 mmol), compound **5a** (270 mg, 1.7 mmol)and acetic acid (360 mg, 6 mmol) were added to 4 mL acetonitrile, and stirred at 70-80 °C for 7-8 hours. After the reaction was completed, it was then washed with sodium bicarbonate aqueous solution, extracted with ethyl acetate, dried over anhydrous sodium sulfate, and concentrated under reduced pressureto obtain intermediate **5c,** which was directly used for the next reaction. MS m/z (ESI): 504.2 $[M+1]^+$.

Step 2: Intermediate **5c** was added to anhydrous THF, LiBr (4.0 equiv.) was added, and stirred at 70-80 °C for

21 hours. After the reaction was completed, it was then quenched with 0.5M sulfuric acid solution, extrated withethyl acetate, washed with sodium bicarbonate solution, dried over anhydrous sodium sulfate, and concentratedto obtain a mixture. The mixture was purified by silica gel column chromatography, and eluted gradiently with dichloromethane/methanol to obtain Compound **4**(pale yellow solid, 72 mg, two-step yield 14.7%). MS m/z (ESI): 490.2 [M+1]$^+$.

$^1$H NMR (400 MHz, CDC13) δ 12.38 (br s, 1H), 10.37 (t, J=5.9 Hz, 1H), 8.38 (s, 1H), 7.38-7.32 (m, 1H), 6.84-6.77 (m, 2H), 5.21 (t, J=5.2 Hz, 1H), 4.91 (q, J=7.0 Hz, 1H), 4.65 (dd, J=16.3, 5.1 Hz, 1H), 4.61 (dd, J=16.6, 5.1 Hz, 1H), 4.17 (dd, J=13.7, 4.1 Hz, 1H), 3.98 (d, J=12.18 Hz, 1H, 3.88 (d, J=12.1 Hz, 1H), 3.80-3.55 (m, 4H), 1.78-1.72 (m, 1H), 1.64-1.59 (m, 1H), 1.54-1.48 (m, 1H), 1.34 (d, J=7.1 Hz, 3H), 1.32-1.29 (m, 1H).

Example 6

An example of the present invention provides a polycyclic carbamoyl pyridone derivative having the structural formula as follows:

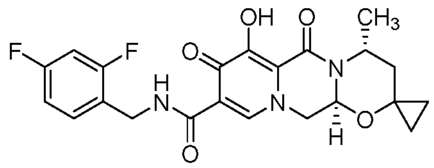
;

named:(4'R, 12a'S)-N-(2,4-difluorophenyl)-7'-hydroxy-4'-methyl-6',8'-dioxo-3',4',6',8', 12', 12a' -hexahydrospiro[cyclopropane-1,2'-pyrido[1',2':4,5]pyrazine[2,1-b][1,3]oxazine]-9'-formami de.

The present example provides a preparation method for the above-mentioned polycyclic carbamoyl pyridone derivative, comprising:

Preparation of intermediate **6a:**

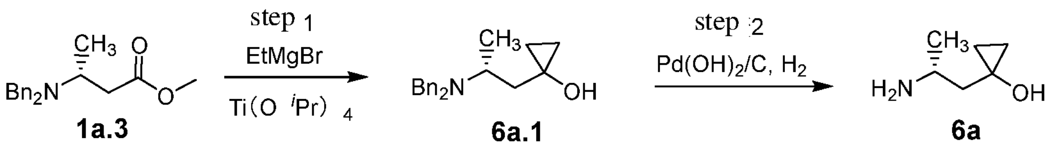

**1a.3** **6a.1** **6a**
;

Step 1: Compound **1a.3** (500 mg, 1.7 mmol) was added to anhydrous THF (10 mL), and cooled down to -30 °C under nitrogen protection. Ti(OiPr)$_4$ (0.25 equiv.) and EtMgbrs (3M/Et$_2$O, 3.0 equiv.) were added dropwise sequentially, and the reaction was stirred at room temperature for 10-12 hours. After the reaction was completed, it was then quenched with ammonium chloride solution, extracted with ethyl acetate, washed with sodium chloride solution, dried over anhydrous sodium sulfate, and concentrated under reduced pressure. And then separated by aluminum trioxide column chromatography, eluted with ethyl acetate and petroleum ether (1:9) to obtain intermediate **6a.1** (colorless oily substance, 254 mg, 51%).

$^1$H NMR (400 MHz, CDC13) δ 7.40-7.19 (m, 10H), 6.78 (s, 1H), 3.99 (d, J=12.9 Hz, 2H), 3.38-3.24 (m, 1H), 3.28 (d, J=12.9 Hz, 2H), 2.52 (ddd, J=14.3, 12.4, 1.9 Hz, 1H), 1.07 (d, J=6.7 Hz, 3H), 0.77 (dd, J=14.6, 2.9 Hz, 1H), 0.74-0.65 (m, 1H), 0.39-0.22 (m, 2H), 0.01-(-0.10) (m, 1H); 13C NMR (100 MHz, CDC13) δ 138.5, 129.5, 128.5, 127.4, 56.6, 53.6, 53.3, 40.1, 12.7, 12.6, 12.0.

Step 2: Referring to the reaction of deprotection of Bn in step 6 for compound **1a,** compound **6a** (a colorless oily substance, 390 mg,-100%) was obtained.

$^1$H NMR (400 MHz, CDC13) δ: 3.46 (s, 1H), 3.35-3.19 (m, 1H), 1.80 (ddd, J=14.4, 9.9, 1.7 Hz, 1H), 1.28-1.22 (m, 1H), 1.17 (d, J=6.4 Hz, 3H), 0.82-0.73 (m, 1H), 0.73-0.65 (m, 1H), 0.50-0.38 (m, 1H), 0.38-0.29 (m, 1H).

Preparation of compound **6:**

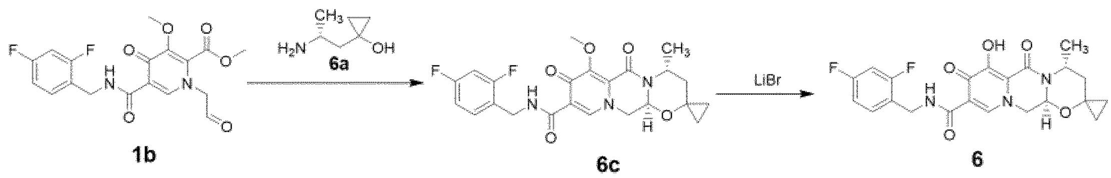

1b 6c 6

;

Step 1: Compound **1b** (268 mg, 0.65 mmol), compound **6a** (150 mg, 1.3 mmol) and acetic acid (234 mg, 3.9 mmol) were added to 3 mL acetonitrile, and stirred at 70-80 °C for 6-8 hours. After the reaction was completed, it was then washed with sodium bicarbonate aqueous solution, extracted with ethyl acetate, dried over anhydrous sodium sulfate, and concentrated under reduced pressureto obtain intermediate **6c,** which was directly used for the next reaction. MS m/z (ESI): 460.2 $[M+1]^+$.

Step 2: Intermediate **6c** was added to anhydrous THF, LiBr (4.0 equiv.) was added, and stirred at 70-80 °C for 14 hours. After the reaction was completed, it was then quenched with 0.5M sulfuric acid solution, extracted with ethyl acetate, washed with sodium bicarbonate solution, dried over anhydrous sodium sulfate, and concentrated to obtain a mixture. The mixture was purified by silica gel column chromatography, and eluted gradiently with dichloromethane/methanol to obtainCompound **4** (faint yellow solid, 110 mg, two-step yield 38%). MS m/z (ESI): 446.2 $[M+1]^+$.

$^1$H NMR (400 MHz, CDC13) δ 12.50 (br s, 1H), 10.39 (t, J=5.8 Hz, 1H), 8.36 (s, 1H), 7.38-7.32 (m, 1H), 6.84-6.76 (m, 2H), 5.21 (t, J=5.2 Hz, 1H), 5.08-5.01 (m, 1H), 4.63 (dd, J=16.3, 6.2 Hz, 1H), 4.59 (dd, J=16.0, 6.1 Hz, 1H), 4.35 (dd, J=13.4, 3.0 Hz, 1H), 4.15 (dd, J=12.5), 5.0 Hz, 1H, 2.62 (dd, J=13.1, 5.2 Hz, 1H), 1.49 (d, J=7.1 Hz, 3H), 1.13 (dd, J=13.9, 1.1 Hz, 1H), 1.05-0.98 (m, 1H), 0.88-0.82 (m, 1H), 0.70-0.64 (m, 1H), 0.56-0.50 (m, 1H).

Example 7

Anexample of the present invention provides a polycyclic carbamoyl pyridone derivative having the structural formula as follows:

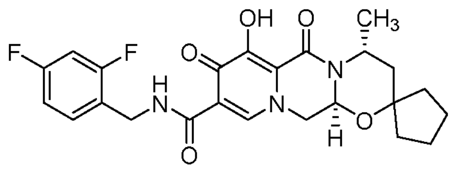

;

named:(4'R, 12a'S)-N-(2,4-difluorophenyl)-7'-hydroxy-4'-methyl-6',8'-oxo-3',4',6',8', 12', 12a'-h exahydrospiro[cyclopentane-1,2'-pyridino[1',2':4,5]pyrazine[2,1-b] [1,3]oxazine]-9'-formamid e.

The present example provides a preparation method for the above-mentioned polycyclic carbamoyl pyridone derivative, comprising:

Preparation of intermediate **7a:**

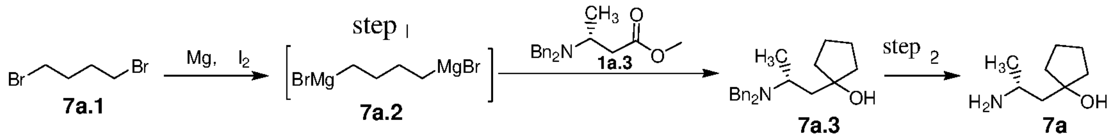

7a.1 7a.2 7a.3 7a

;

Step 1: Magnesium chips (14.59 g, 600 mmol), iodine particles (0.91 g, 3.6 mmol) and 50 mL anhydrous THF were added under a nitrogen gas atmosphere. Heated up to 60 °C, and 1,2-dibromoethane (0.1 mL) was added dropwise quickly, then 1,4-dibromobutane (14.3 mL, 120 mmol) was added dropwise. After the addition was completed, the reaction was stirred for 1 hour. After the reaction was completed, temperature was raised to 80 °C, intermediate **1a.3** (8.92 g dissolved in 20 mL anhydrous THF, 30 mmol) was added, and the reaction was stirred at this temperature for additional 2 hours. After the reaction was completed, it was then quenched with ammonium chloride solution, filtered, the filtrate was extracted with ethyl acetate, washed with sodium chloride solution, dried over anhydrous sodium sulfate, and concentrated under reduced pressure rotary evaporation to obtain a mixture.The mixture was separated by silica

gel column chromatography, eluted with ethyl acetate and petroleum ether to obtain intermediate 7**a.3** (faint yellow oily substance 4.36 g, 45%).

$^1$H NMR (400 MHz, CDC13) δ 7.34-7.29 (m, 8H), 7.28-7.22 (m, 2H), 6.70 (s, 1H), 3.93 (d, J=12.8 Hz, 2H), 3.29-3.21 (m, 1H), 3.21 (d, J=12.8 Hz, 2H), 2.18 (dd, J=14.6, 11.8 Hz, 1H), 1.78-1.62 (m, 2H), 1.60-1.51 (m, 1H), 1.47-1.33 (m, 3H), 1.13 (dd, J=14.6, 2.4 Hz, 1H), 1.03 8 (d, J=6.7 Hz, 3H), 0.95-0.84 (m, 1H), 0.80-0.71 (m, 1H) 13C NMR (100 MHz, CDCl3) δ 138.39, 129.76, 128.45, 127.33, 82.41, 52.88, 51.46, 42.37, 41.53, 38.08, 23.96, 23.47, 12.39.

Step 2: Referring to the de-Bn synthesis operation for **1a**, a white solid intermediate **7a** was obtained and directly used for the next reaction.

$^1$H NMR (400 MHz, CDC13) δ 3.21-3.08 (m, 1H), 1.88-1.40 (m, 10H), 1.13 (d, J=6.4 Hz, 3H); 13C NMR (100 MHz, CDC13) δ 82.2, 47.4, 46.5, 41.7, 39.2, 28.2, 24.1, 23.7.

Preparation of compound **7:**

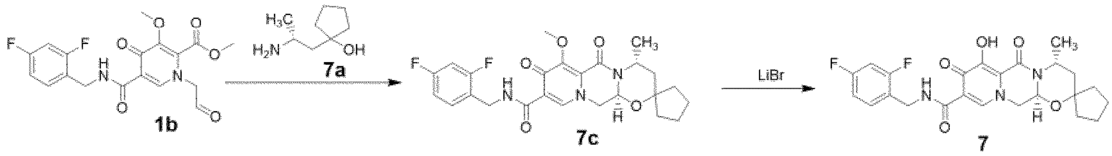


1b 7c 7

;

Step 1: Compound **1b** (346 mg, 0.84 mmol), compound **7a** (300 mg, 2.1 mmol) and acetic acid (403 mg, 7.72 mmol) were added to 4 mL acetonitrile, and stirred at 70-80 °C for 6 hours. After the reaction was completed, it was then washed with sodium bicarbonate aqueous solution, extracted with ethyl acetate, dried over anhydrous sodium sulfate, and concentrated under reduced pressure to obtain intermediate **7c,** which was directly used for the next reaction. MS m/z (ESI): 488.2 $[M+1]^+$.

Step 2: Intermediate **7c** was added to anhydrous THF, LiBr (4.0 equiv.) was added, and stirred at 70-80 °C for 6 hours. After the reaction was completed, it was then quenched with 0.5M sulfuric acid solution,extractedwith ethyl acetate, washed with sodium bicarbonate solution, dried over anhydrous sodium sulfate, and concentrated to obtain a mixture. The mixture was purified to silica gel column chromatography, and eluted gradiently with dichloromethane/methanolto obtain Compound **4** (pale yellow solid, 70 mg, two-step yield 17.6%). MS m/z (ESI): 474.2 $[M+1]^+$.

$^1$H NMR (400 MHz, CDC13) δ 12.47 (br s, 1H), 10.42 (t, J=5.8 Hz, 1H), 8.34 (s, 1H), 7.40-7.34 (m, 1H), 6.84-6.77 (m, 2H), 5.35 (t, J=3.4 Hz, 1H), 4.83-4.75 (m, 1H), 4.66 (dd, J=15.3, 6.0 Hz, 1H), 4.60 (dd, J=15.3, 5.8 Hz, 1H), 4.30 (dd, J=13.7, 3.4 Hz, 1H), 4.17 (dd, J=13.7 3.5 Hz, 1H, 2.11 (dd, J=14.5, 6.8 Hz, 1H), 2.07-1.89 (m, 2H), 1.83 (dd, J=14.5, 8.1 Hz, 1H), 1.80-1.49 (m, 6H), 1.39 (d, J=6.8 Hz, 3H).

Example 8

An example of the present invention provides a polycyclic carbamoyl pyridone derivative having the structural formula as follows:

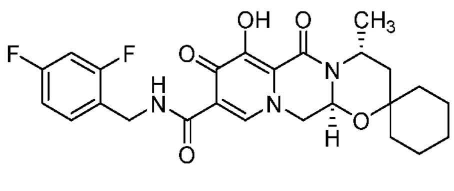

;

named:(4'R,12a'S)-N-(2,4-difluorophenyl)-7'-hydroxy-4'-methyl-6',8'-oxo-3',4',6',8',12',12a'-h exahydrospiro[cyclohexane-1,2'-pyridino[1',2':4,5]pyrazine[2,1-b][1,3]oxazine]-9'-formamid e.

The present example provides a preparation method for the above-mentioned polycyclic carbamoyl pyridone derivative, comprising:

Preparation of intermediate **8a:**

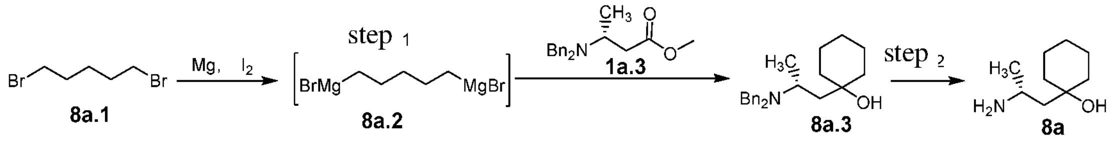


8a.1 8a.2 1a.3 8a.3 8a ;

Step 1: Referring to the operating conditions for intermediate **7a.3,** intermediate **8a.3** (white solid, 2.68g, 16%) was prepared with 1,5-dibromopentane.

$^1$H NMR (400 MHz, CDC13) δ 7.35-7.30 (m, 8H), 7.27-7.21 (m, 2H), 6.62 (s, 1H), 3.92 (d, J=12.8 Hz, 2H), 3.24-3.13 (m, 1H), 3.19 (d, J=12.8 Hz, 2H), 1.81 (dd, J=14.8, 12.0 Hz, 1H), 1.70-1.58 (m, 1H), 1.52 (dd, J=12.9, 5.6 Hz, 1H), 1.46-1.32 (m, 3H), 1.25-1.08 (m, 4H), 1.05 (d, J=6.7 Hz, 3H), 0.71 (dd, J=7.3, 4.8 Hz, 2H) 13C NMR (100 MHz, CDC13) δ 138.50, 129.90, 128.52, 127.40, 71.51, 52.95, 49.18, 42.67, 40.60, 35.77, 26.14, 22.24, 22.13, 12.52

Step 2: Referring to the de-Bn peration conditions for inintermediate **1a,** a white solid intermediate **8a** was prepared and directly used for the next reaction.

Synthesis of compound **8:**

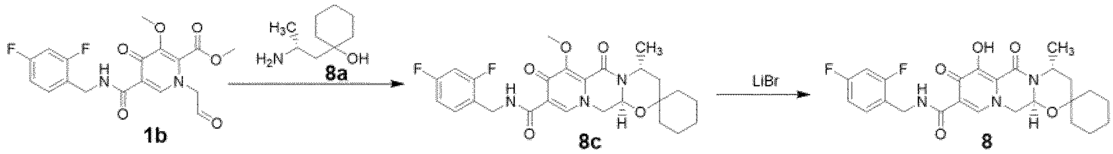


1b 8a 8c 8 ;

Step 1: Compound **1b** (412 mg, 1 mmol), compound **8a**(300 mg, 2.2 mmol)and acetic acid (360 mg, 6 mmol) were added to 4 mL acetonitrile, and stirred at 70-80 °C for 16 hours. After the reaction was completed, it was then washed with sodium bicarbonate aqueous solution, extracted with ethyl acetate, dried over anhydrous sodium sulfate, and concentrated under reduced pressure to obtain intermediate **7c,** which was directly used for the next reaction. MS m/z (ESI): 502.2 $[M+1]^+$.

Step 2: Intermediate **8c** was added to anhydrous THF, LiBr (4.0 equiv.) was added, and stirred at 70-80 °C for 6 hours. After the reaction was completed, it was then quenched with 0.5M sulfuric acid solution, extracted with ethyl acetate, washed with sodium bicarbonate solution, dried over anhydrous sodium sulfate, and concentrated to obtain a mixture. The mixture was pruified by silica gel column chromatography, and eluted gradiently with dichloromethane/methanol to obtain Compound **8** (pale yellow solid, 194 mg, two-step yield 40.0%). MS m/z (ESI): 488.2 $[M+1]^+$.

$^1$H NMR (400 MHz, CDC13) δ 12.40 (br s, 1H), 10.48 (t, J=5.9 Hz, 1H), 8.42 (s, 1H), 7.40-7.34 (m, 1H), 6.85-6.77 (m, 2H), 5.35 (t, J=2.5 Hz, 1H), 4.70-4.59 (m, 3H), 4.28 (dd, J=13.9, 3.1 Hz, 2H), 2.04 (dd, J=15.0, 6.9 Hz, 1H), 1.80 (dd, J=15.0, 10.4 Hz, 1H), 1.68-1.54 (m, 2H) H), 1.44-1.21 (m, 8H), 1.35 (d, J=6.5 Hz, 3H).

Example 9

An example of the present invention provides a polycyclic carbamoyl pyridone derivative having the structural formula as follows:

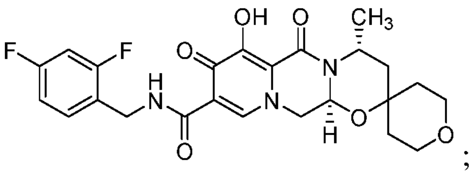
;

named:(4R,12aS)-N-(2,4-difluorophenyl)-7'-hydroxy-4'-methyl-6',8'-dioxo-2,3,3',4',5,6,6',8',1 2',12a'-decahydrospiro[pyran-4,2'-pyridino[1',2':4,5]pyrazine[2,1-b][1,3]oxazine]-9'-formami de.

The present example provides a preparation method for the above-mentioned polycyclic carbamoyl pyridone derivative, comprising:

Preparation of intermediate **9a:**

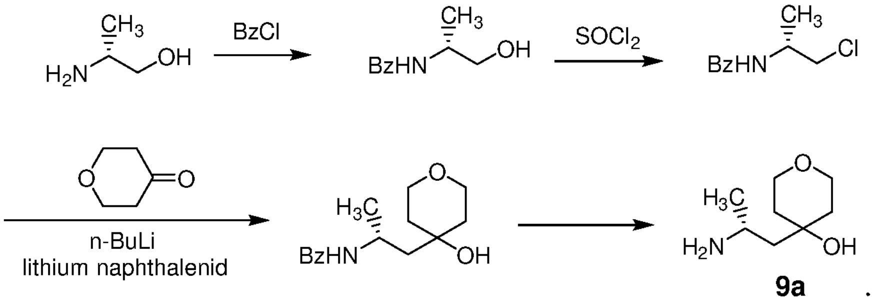


**9a** ;

Step: The preparation method refers to Tetrahedron Asymmetry 1996, 2911-2922. (R) -2-aminopropanol was used as raw material,then subjected to amino Bz-protection and hydroxyl-chlorinated, and followed by the addition reaction on tetrahydropyranone in n-BuLi/Lithium naphthalenid system, and finally the intermediate **9a** was obtained by deprotection of Bz. MS m/z (ESI): 160.23 $[M+1]^+$.
$^1$H NMR (DMSO-d6) δ 3.64-3.48 (m, 4H), 3.53-3.36 (m, 3H), 3.12-2.85 (m, 1H), 1.51-1.26 (m, 6H), 1.02 (d, J=6.4 Hz, 3H);

Synthesis of compound **9:**

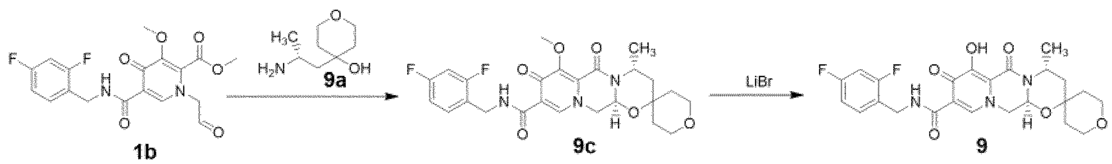


**1b** **9a** **9c** **9** ;

Step 1: Compound **1b** (412 mg, 1 mmol), compound **9a** (318 mg, 2 mmol) and acetic acid (360 mg, 6 mmol) were added to 4 mL acetonitrile, and stirred at 70-80 °C for 10-12 hours. After the reaction was completed, it was then washed with sodium bicarbonate aqueous solution, extracted with ethyl acetate, dried over anhydrous sodium sulfate, and concentrated under reduced pressure to obtain intermediate **9c,** which was directly used for the next reaction. MS m/z (ESI): 504.2 $[M+1]^+$.

Step 2: Intermediate **9c** was added to anhydrous THF, LiBr (4.0 equiv.) was added, and stirred at 70-80 °C for 6-8 hours. After the reaction was completed, it was then quenched with 0.5M sulfuric acid solution, extracted with ethyl acetate, washed with sodium bicarbonate solution, dried over anhydrous sodium sulfate, and concentrated to obtain a mixture. The mixture was purified by silica gel column chromatography, and eluted gradiently with dichloromethane/methanolto obtain Compound **9** (pale yellow solid, 68 mg, two-step yield 13.9%). MS m/z (ESI): 490.2 $[M+1]^+$.

$^1$H NMR (400 MHz, CDC13) δ 12.31 (br s, 1H), 10.40 (t, J=5.8 Hz, 1H), 8.36 (s, 1H), 7.40-7.34 (m, 1H), 6.85-6.77 (m, 2H), 5.39 (t, J=2.1 Hz, 1H), 4.74-4.60 (m, 3H), 4.31 (dd, J=13.9, 3.0 Hz, 1H), 4.24 (dd, J=14.0, 1.9 Hz, 1H), 3.72-3.65 (m, 2H), 3.60 (dt, J=11.6, 4.2 Hz, 1H) 3.48 (td, J=11.1, 2.6 Hz, 1H), 2.04 (dd, J=15.0, 6.9 Hz, 1H), 1.90 (dd, J=15.0, 10.3 Hz, 1H), 1.74-1.62 (m, 2H), 1.59 (dd, J=10.8, 4.7 Hz, 1H), 1.55 (dd, J=10.8, 4.6 Hz, 1H), 1.39 (d, J=6.5 Hz, 3H).

Example 10

Anexample of the present invention provides a polycyclic carbamoyl pyridone derivative having the structural formulaas follows:

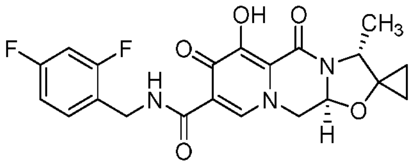

,

named: (3R,11aS)-N-(2,4-difluorophenyl)-6-hydroxy-3-methyl-5,7-dioxo-5,7,11,11a-tetrahydro-3H-s piro[cyclopropane-1,2-oxazole[3,2-a]pyridino[1,2-d]pyrazine]-8-formamide.

The present example provides a preparation method for the above-mentioned polycyclic carbamoyl pyridone derivative, comprising:

Preparation of intermediate **10a:**

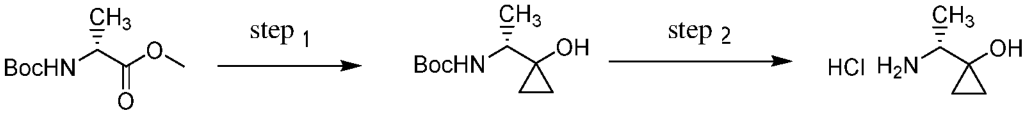


**10a.1** **10a.2** **10a** ;

Step 1: Under a nitrogen gas atmosphere, compound **1a.1** (4.06 g, 20 mmol) was added to 40 mL anhydrous THF, cooled down to -30--35 °C, and $Ti(OiPr)_4$ (0.3 equiv.) and EtMgBr s (2M/$Et_2O$, 3.0 equiv.) were added dropwise. After the reaction was stirred for 1 hour, transferred to -5-0 °C and stirred for 10-12 hours. After the reaction was completed, it was then quenched with ammonium chloride solution, extracted with ethyl acetate, washed with sodium chloride solution, dried over anhydrous sodium sulfate, and concentrated under reduced pressure, separated by silica gel column chromatography, eluted gradiently with ethyl acetate and petroleum ether to obtain intermediate **10a.2** (a white solid 0.6 g, 15%).

$^1H$ NMR (400 MHz, CDC13) δ 3.31 (q, J=6.8 Hz, 1H), 1.45 (s, 9H), 1.23 (d, J=6.9 Hz, 3H), 0.82-0.72 (m, 3H), 0.54-0.51 (s, 1H).

Step 2: Intermediate **10a.2** (390 mg, 1.94 mmol) was added to 4 mL dioxane, 3M hydrochloric acid dioxane solution (7.76 mmol) (preparation of 3M hydrochloric acid: 1 mL of concentrated hydrochloric acid was added to 3 mL dioxane)was added dropwise, and the reaction was stirred at room temperature for 20-22 hours. After the reaction was completed, the reaction solution was concentrated under reduced pressure, water-carried with acetonitrile multiple times to obtain a pink oily substance, then 4 mL acetonitrile was added and refrigerated for 10-12 hours, filtered, washed with cold acetonitrile, and dried to obtain intermediate **10a** (white solid 180 mg, 91.8%).

$^1H$ NMR (400 MHz, DMSO-d6) δ 8.15 (br s, 3H), 5.81 (br s, 1H), 2.79-2.74 (m, 1H), 1.23 (d, J=6.7 Hz, 3H), 0.72-0.54 (m, 4H).

Synthesis of compound **10:**

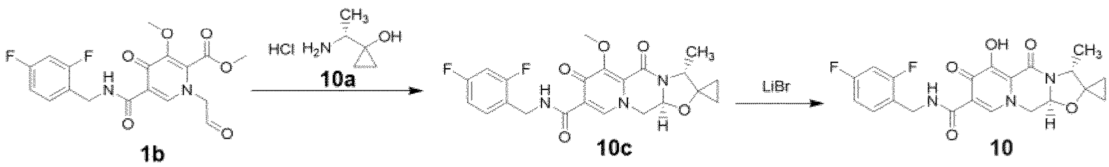


**1b** **10c** **10** ;

Step 1: Compound **1b** (206 mg, 0.5 mmol) was added to 3 mL acetonitrile solution, while DIEA (130 mg, 1 mmol) was added to a acetonitrile solution (2 mL)of another compound **10a** hydrochloride (138 mg, 1 mmol), after mixied uniformly, the mixture solution was added to the acetonitrile solution of compound **1b**, and finally acetic acid (150 mg, 2.5 mmol) was added, and the mixture was stirred at 70-80 °C for 4-5 hours. After the reaction was completed, it was then washed with sodium bicarbonate aqueous solution, extracted with ethyl acetate, dried over anhydrous sodium sulfate, and concentrated under reduced pressure to obtainintermediate **10c,** which was directly used for the next

reaction. MS m/z (ESI): 446.2 $[M+1]^+$.

Step 2: Intermediate **10c** was added to anhydrous THF, LiBr (4.0 equiv.) was added, and the reaction was stirred at 70-80 °C for 6-8 hours. After the reaction was completed, it was then quenched with 0.5M sulfuric acid solution, extracted with ethyl acetate, washed with sodium bicarbonate solution, dried over anhydrous sodium sulfate, and concentrated to obtain a mixture. The mixture was purified by silica gel column chromatography, and eluted gradiently with dichloromethane/methanolto obtain Compound **10** (an off white solid, 142 mg, two-step yield 66%). MS m/z (ESI): 432.2 $[M+1]^+$.

$^1H$ NMR (400 MHz, CDC13) δ 11.47 (br s, 1H), 10.31 (s, 1H), 8.36 (s, 1H), 7.38-7.32 (m, 1H), 6.83-6.78 (m, 2H), 5.41-5.39 (m, 1H), 4.68-4.56 (m, 1H), 4.45 (dd, J=12.3, 3.0 Hz, 1H), 4.35 (q, J=6.1 Hz, 1H), 3.98 (t, J=11.2 Hz, 1H), 1.34 (d, J=6.2 Hz, 3H), 1.25-1.10 (m, 1H), 0.94-0.76 (m, 3H).

Example 11

An example of the present invention provides a polycyclic carbamoyl pyridone derivative having the structural formula as follow:

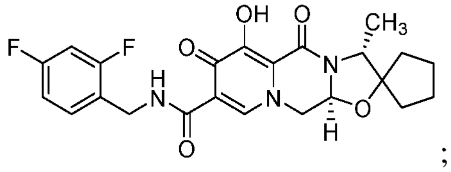
;

named: (3'R,11a'S)-N-(2,4-difluorophenyl)-6'-hydroxy-3'-methyl-5',7'-dioxo-5',7',11',11a'-tetrahydro-3'H-spiro[cyclopentane-1,2'-oxazole[3,2-a]pyridino[1,2-d]pyrazine]-8'-formamide.

The present example provides a preparation method for the above-mentioned polycyclic carbamoyl pyridone derivative, comprising:

Preparation of intermediate **11a:**

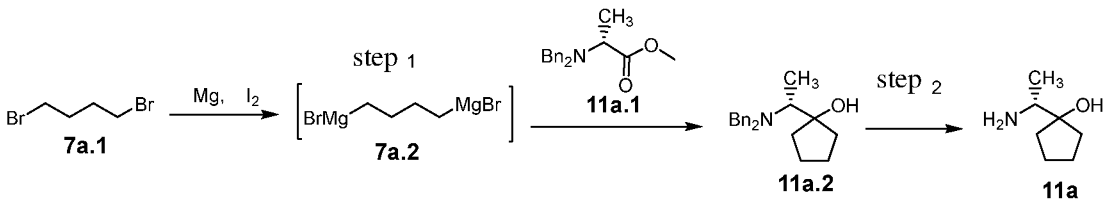

7a.1 7a.2 11a.1 11a.2 11a ;

Step 1: Referring to the operating conditions for **7a.3**, intermediate **11a.1** was added to the freshly prepared Grignard reaction solution **7a.2** to prepare intermediate **11a.2** (3.91g of pale yellow oily substance, 50%).

$^1H$ NMR (400 MHz, CDC13) δ 7.35-7.27 (m, 8H), 7.26-7.21 (m, 2H), 4.35 (s, 1H), 3.89 (d, J=13.5 Hz, 2H), 3.34 (d, J=13.5 Hz, 2H), 2.92 (q, J=7.0 Hz, 1H), 1.90-1.78 (m, 1H), 1.78-1.68 (m, 1H), 1.69-1.54 (m, 3H), 1.52-1.38 (m, 3H), 1.09 (d, J=7.0 Hz, 3H) 13C NMR (100 MHz, CDC13) δ 139.53, 129.09, 128.55, 127.30, 82.63, 59.33, 55.09, 38.98, 35.53, 24.89, 23.12, 7.13.

Step 2: Referring to the de-Bn operating conditions for **1a,** the oily substance **11a** was prepared and directly used for the next reaction.

Synthesis of compound **11:**

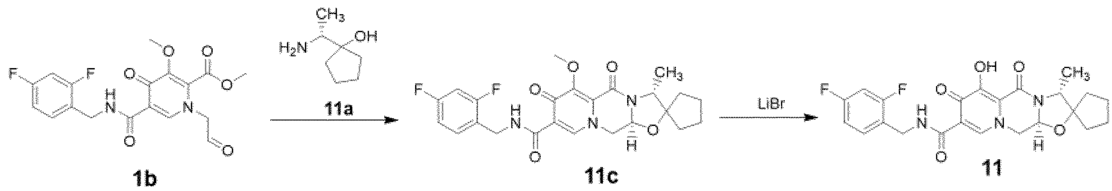

1b
11c
11
;

Step 1: Compound **1b** (251 mg, 0.84 mmol), compound **7a** (160 mg, 1.22 mmol) and acetic acid (220 mg, 3.66 mmol) were added to 3 mL acetonitrile, and stirred at 70-80 °C for 16 hours. After the reaction was completed, it was then washed with sodium bicarbonate aqueous solution, extracted with ethyl acetate, dried over anhydrous sodium sulfate, and concentrated under reduced pressure to obtain intermediate **11c,** which was directly used for the next reaction. MS m/z (ESI): 474.2 $[M+1]^+$.

Step 2: Intermediate **11c** was added to anhydrous THF, LiBr (4.0 equiv.) was added, and stirred at 70-80 °C for 6-8 hours. After the reaction was completed, it was then quenched with 0.5M sulfuric acid solution, 3-5 mL water was added and stirred to precipitate the solid, then filtered, washed with THF/water = 1:1, and dried to obtain compound **11** (white solid, 230 mg, two-step yield 63.5%). MS m/z (ESI): 432.2 $[M+1]^+$.

$^1$H NMR (400 MHz, DMSO-d6) δ 11.44 (br s, 1H), 10.33 (t, J=3.6Hz, 1H), 8.46 (s, 1H), 7.43-7.37 (m, 1H), 7.28-7.22 (m, 1H), 7.09-7.04 (m, 1H), 5.42 (d, J=10.2, 4.0 Hz, 1H), 4.83 (d, J=12.2, 4.0 Hz, 1H), 4.59-4.50 (m, 2H), 4.17 (q, J=6.5 Hz, 1H), 3.99 (dd, J=11.6, 10.44 Hz, 1H), 1.79 9-1.59 (m, 8H), 1.30 (d, J=6.7 Hz, 3H).

Example 12

An example of the present invention provides a polycyclic carbamoyl pyridone derivative having the structural formula as follow:

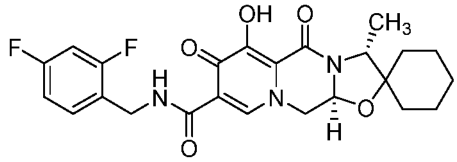
;

named: (3'R,11a'S)-N-(2,4-difluorophenyl)-6'-hydroxy-3'-methyl-5',7'-dioxo-5',7',11',11a'-tetrahydro-3'H-spiro[cyclohexane-1,2'-oxazole[3,2-a]pyridino[1,2-d]pyrazine]-8'-formamide.

The present example provides a preparation method for the above-mentioned polycyclic carbamoyl pyridone derivative, comprising:

Preparation of intermediate **12a:**

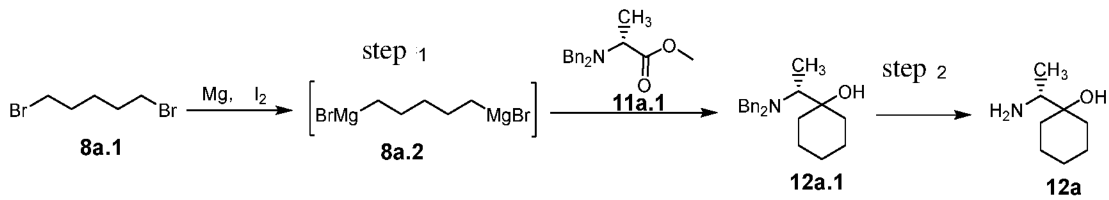

8a.1
8a.2
12a.1
12a
;

Step 1: Referring to the operating conditions for **7a.3,** intermediate **11a.1** was used for Grignard reaction to prepare intermediate **12a.1** (white solid, 2.03 g, 12%).

$^1$H NMR (400 MHz, CDC13) δ 7.34-7.29 (m, 7H), 7.28-7.21 (m, 1H), 3.89 (d, J=13.7 Hz, 2H), 3.71 (s, 1H), 3.36 (d, J=13.7 Hz, 2H), 2.61 (q, J=7.1 Hz, 1H), 1.73-1.18 (m, 10H), 1.09 (d, J=7.1 Hz, 3H) 13C NMR (100 MHz, CDC13) δ 139.62, 129.10, 128.53, 127.28, 72.56, 62.38, 56.18, 36.32, 33.35, 26.05, 22.42, 21.96, 7.50

Step 2: Referring to the de-Bn operating conditions for **1a**, an oily substance **12a** was prepared and directly used for the next reaction.

Synthesis of compound **12:**

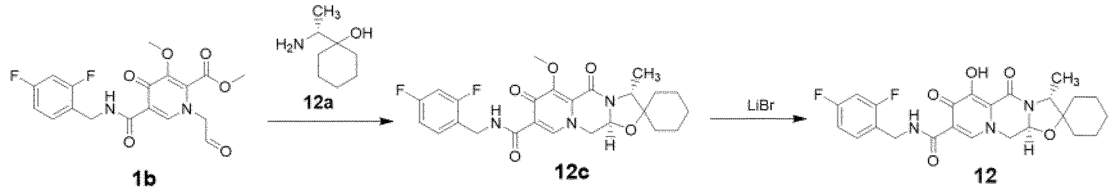


1b 12c 12 ;

Step 1: Compound **1b** (231 mg, 0.56 mmol), compound **12a** (200 mg, 1.4 mmol) and acetic acid (202 mg, 3.36 mmol) were added to 3 mL acetonitrile, and stirred at 70-80 °C for 4-5 hours. After the reaction was completed, it was then washed with sodium bicarbonate aqueous solution, extracted with ethyl acetate, dried over anhydrous sodium sulfate, filtered, and concentrated under reduced pressure to obtain intermediate **11a,** which was directly used for the next reaction. MS m/z (ESI): 488.2 $[M+1]^+$.

Step 2: Intermediate **11c** was added to anhydrous THF, LiBr (4.0 equiv.) was added, and stirred at 70-80 °C for 10-12 hours. After the reaction was completed, it was then quenched with 0.5M sulfuric acid solution, extracted with ethyl acetate, washed with sodium bicarbonate solution, dried overanhydrous sodium sulfate, filtered, and concentrated to obtain a mixture, and then methyl tert butyl ether was added and stirred for crystallization for 2-4 hours, filtrated, washed with methyl tert butyl ether, and dried to obtain compound **12** (an off-white solid, 180 mg, two-step yield 67.9%). MS m/z (ESI): 474.2 $[M+1]^+$.

$^1$H NMR (400 MHz, CDC13) δ 11.55 (br s, 1H), 10.33 (t, J=5.7 Hz, 1H), 8.35 (s, 1H), 7.39-7.32 (m, 1H), 6.84-6.77 (m, 2H), 5.40 (dd, J=10.1, 4.0 Hz, 1H), 4.65 (dd, J=16.0, 6.2 Hz, 1H), 4.61 (dd, J=16.2, 6.2 Hz, 1H), 4.42 (dd, J=12.4, 4.2 Hz, 1H), 4.13 (q, J=6.7 Hz, 1H), 3.87 (dd J=12.3, 10.3 Hz, 1H), 1.80-1.50 (m, 10H), 1.36 (d, J=6.8 Hz, 3H).

Example 13

An example of the present invention provides polycyclic carbamoyl pyridone derivatives having the structural formulaas follow:

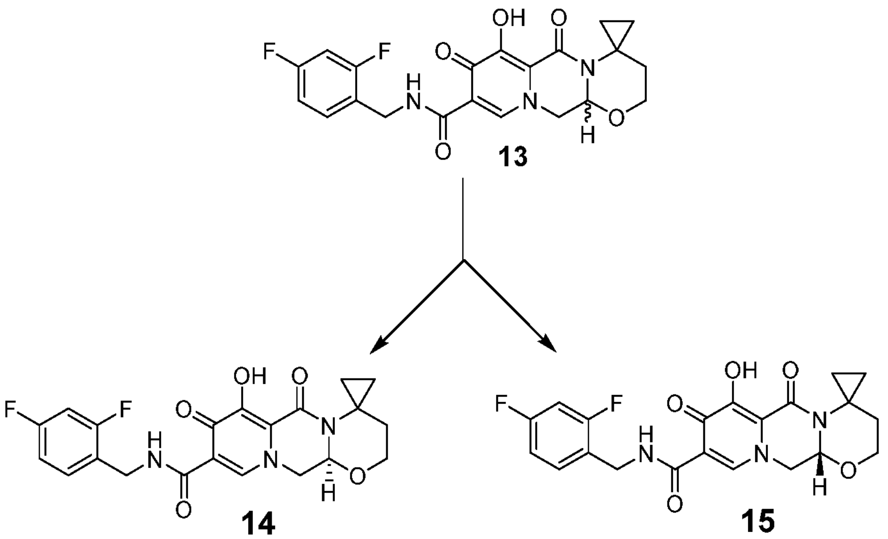

13

14 15 ,

named:

(2,4-difluorophenyl)-7'-hydroxy-6',8'-dioxo-2',3',6',8',12',12a'-hexahydrospiro [cycloprop ane-1,4'-pyridino[1',2':4,5]pyrazine[2,1-b] [1,3]oxazine]-9'-formamide(compoundl3);

(S)-N-(2,4-difluorophenyl)-7'-hydroxy-6',8'-dioxo-2',3',6',8',12',12a'-hexahydrospiro[cyc lopropane-1,4'-pyridino[1',2':4,5]pyrazine[2,1-b][1,3]oxazine]-9'-formamide(compound14);

(R)-N-(2,4-difluorophenyl)-7'-hydroxy-6',8'-dioxo-2',3',6',8',12',12a'-hexahydrospiro[cyc lopropane-1,4'-pyridino[1',2':4,5]pyrazine[2,1-b][1,3]oxazine]-9'-formamide(compound15).

The present example provides a preparation method for the above-mentioned polycyclic carbamoyl pyridone derivatives, comprising:

Preparation of intermediate **13a:**

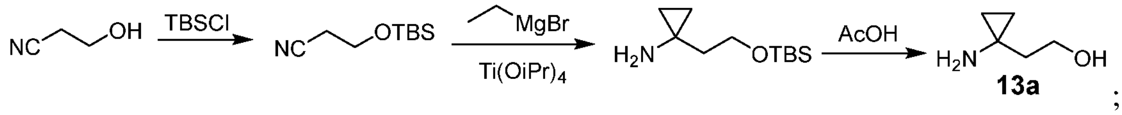


Steps 1 and 2: Referring to the synthesis route of Journal of Medical Chemistry, 2016, 59, 8, 3732-3749, 2-cyanoethanol was used as raw materials, and followed by the reaction of hydroxyl TBSCl-protection and Kulinovich cyclopropane reaction,and then theTBS protected amino alcohols was prepared.

$^1$H NMR (DMSO-d6) δ 3.78 (t, J=6.8 Hz, 1H), 1.65 (br s, 2H), 1.53 (t, J=6.9 Hz, 2H), 0.83 (s, 9H), 0.37-0.30 (m, 3H), 0.04 (s, 6H).

Step 3: After deprotection of TBS with acetic acid, a reddish brown oily substance **13a** was obtained, which was directly used for the next reaction.

Synthesis of compounds **13, 14**and **15:**

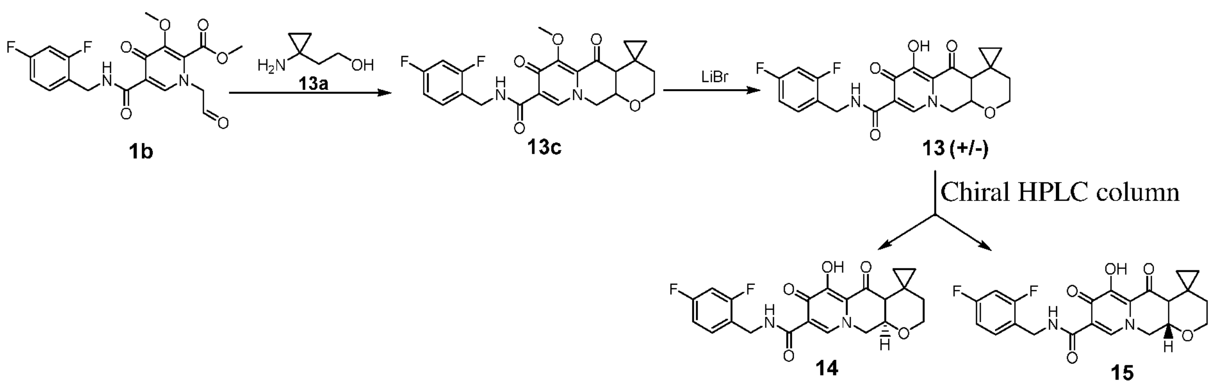


Step 1: Compound **1b** (412 mg, 1 mmol), compound **13a** (202 mg, 2 mmol) and acetic acid (360 mg, 6 mmol) were added to 4 mL acetonitrile, and stirred at 70-80 °C for 14-16 hours. After the reaction was completed, it was then washed with sodium bicarbonate aqueous solution, extracted with ethyl acetate, dried over anhydrous sodium sulfate, and concentrated under reduced pressure to obtain intermediate **13c,** which was directly used for the next reaction. MS m/z (ESI): 446.1 $[M+1]^+$.

Step 2: Intermediate **13c** was added to anhydrous THF, LiBr (4.0 equiv.) was added, and stirred at 70-80 °C for 4-6 hours. After the reaction was completed, it was then quenched with 0.5M sulfuric acid solution, then water was added and stirred for 1-3 hours for crystallization, filtrated, the solid was washed with THF: water=1:1 to obtain compound **13** (an off white solid, 177 mg, two-step yield 41%). MS m/z (ESI): 432.2 $[M+1]^+$.

$^1$H NMR (400 MHz, DMSO-d6) δ 12.53 (br s, 1H), 10.41 (t, J=5.8 Hz, 1H), 8.57 (s, 1H), 7.48-7.42 (m, 1H), 7.33-7.27 (m, 1H), 7.15-7.10 (m, 1H), 5.38-5.34 (d, 1H), 4.74 (dd, J=14.7, 1.2 Hz, 1H), 4.63 (dd, J=15.2, 6.2 Hz, 1H), 4.57 (dd, J=15.0, 5.7 Hz, 1H), 4.43 (dd, J=14.4, 2.94) Hz, 1H, 4.15 (dd, J=11.1, 4.8 Hz, 1H), 4.07 (td, J=11.8, 2.3 Hz, 1H), 2.33 (td, J=12.4, 5.2, 1H), 1.52-1.47 (m, 1H), 1.18-1.08 (m, 2H), 0.88-0.82 (m, 1H), 0.77-0.72 (m, 1H).

Preparation of chiral compounds **14** and **15:**

SFC separation method for racemic mixture**13** (50 mg) (preparation column:ChiralPak AD, 250×30 mm I.D., 10 μm; mobile phase: A-phase $CO_2$/B-phase isopropanol, gradient: B 25%; flow rate: 70 ml/min; column pressure: 100

bar; column temperature: 38 °C, wavelength: 220 nm), prepared compound **14** (retention time: 1.21 min), MS m/z (ESI): 432.1 $[M+1]^+$, and compound **15** (retention time: 1.46 min), m/z (ESI): 432.1 $[M+1]^+$.

Chiral HPLC purity method (preparation column:ChiralPak AD, 150×4.6 mm I.D., 3 μm; mobile phase: A-phase $CO_2$/B-phase isopropanol (0.05% DEA), gradient: B 40%; flow rate: 2.5 ml/min; column pressure: 100 bar; column temperature 35 °C, wavelength 220 nm).

Example 14

Anexample of the present invention provides polycyclic carbamoyl pyridone derivatives having the structural formulaas follow:

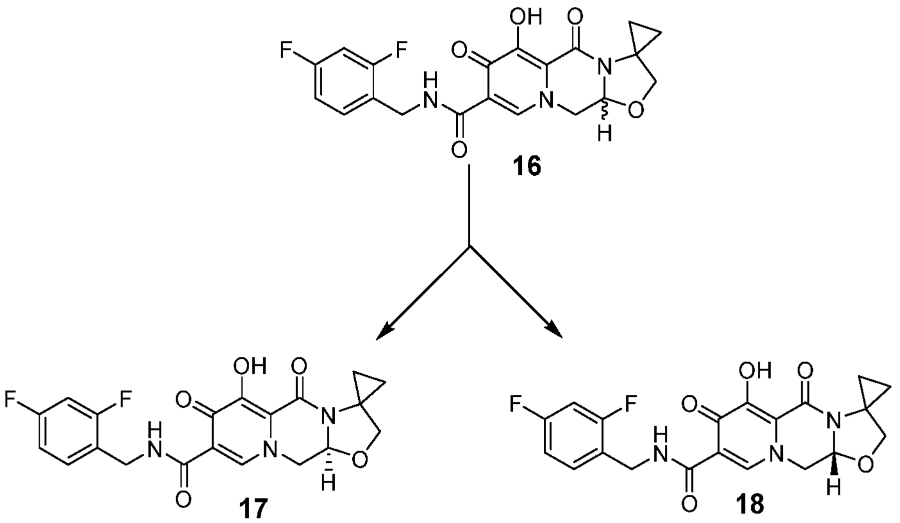

16

17 18 ,

named as follow:

N-(2,4-difluorophenyl)-6'-hydroxy-5',7'-dioxo-5',7',11',11a'-tetrahydro-2'H-spiro[cyclopr opane-1,3'-oxazole[3,2-a]pyridino[1,2-d]pyrazine]-8'-formamide(compound**16**);

(S)-N-(2,4-difluorophenyl)-6'-hydroxy-5',7'-dioxo-5',7',11',11a'-tetrahydro-2'H-spiro[cyc lopropane-1,3'-oxazole[3,2-a]pyridino[1,2-d]pyrazine]-8'-formamide(compound**17**);

(R)-N-(2,4-difluorophenyl)-6'-hydroxy-5',7'-dioxo-5',7',11',11a'-tetrahydro-2'H-spiro[cyc lopropane-1,3'-oxazole[3,2-a]pyridino[1,2-d]pyrazine]-8'-formamide(compound**18**).

The present example provides preparation methods for the above-mentioned polycyclic carbamoyl pyridone derivatives, comprising:

Synthesis of compounds **16, 17**and **18:**

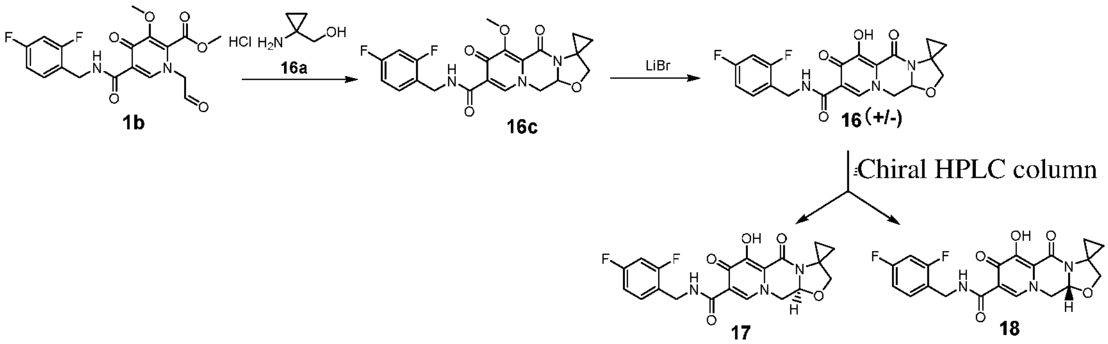


1b 16c 16 (+/-) 17 18 ;

Step 1: Compound **1b** (618 mg, 1.5 mmol) was added to 5 mL acetonitrile solution, while DIEA (387 mg, 3 mmol) was added to 1 mL acetonitrile solution of another compound **16a** hydrochloride (371 mg, 3 mmol), after mixed uniformly, the mixture was added to the acetonitrile solution of compound **1b**, and finally acetic acid (540 mg, 9 mmol) was added, the mixture was stirred at 70-80 °C for 20-24 hours. After the reaction was completed, it was then washed with sodium bicarbonate aqueous solution, extracted with ethyl acetate, dried over anhydrous sodium sulfate, and concentrated under reduced pressure to obtainintermediate **16c,** which was directly used for the next reaction. MS m/z (ESI): 432.2 $[M+1]^+$.

Step 2: Intermediate **16c** was added to anhydrous THF, LiBr (4.0 equiv.) was added, and stirred at 70-80 °C for 6-8 hours. After the reaction was completed, it was then quenched with 0.5M sulfuric acid solution, stirred for 2-4 hours to precipitate the solid, filtered, washed with THF/water ratio of 1:1, and dried to obtaincompound **10** (an off white solid, 230 mg, two-step yield 36.7%). MS m/z (ESI): 418.2 $[M+1]^+$.

$^1H$ NMR (400 MHz, CDC13) δ 11.70 (br s, 1H), 10.25 (s, 1H), 8.27 (s, 1H), 7.31-7.25 (m, 1H), 6.77-6.71 (m, 2H), 5.32 (dd, J=10.1, 3.6 Hz, 1H), 4.56 (d, J=5.8 Hz, 2H), 4.41 (dd, J=12.2, 3.5 Hz, 1H), 4.13 (d, J=8.6 Hz, 1H), 4.06-3.95 (m, 2H), 2.19-2.11 (m, 1H), 2.02-1.94 (m), 1H), 0.83-0.73 (m, 2H).

Preparation of chiral compounds **17** and **18:**

Racemic mixture**16** (100 mg) was separated by SFC (preparation column:ChiralPak AD, 250×30 mm I.D., 10 μm; mobile phase: A phase $CO_2$/B phase isopropanol (0.1% ammonia water), gradient: B 40%; flow rate: 80 ml/min; column pressure: 100 bar; column temperature 38 °C, wavelength 220 nm).

Compounds **17** (51 mg, retention time 2.79 min), MS m/z (ESI): 418.1 [M+1]+, and compound **18** (39 mg, retention time 5.84 min), MS m/z (ESI): 418.1 $[M+1]^+$ were prepared separately.

Chiral HPLC purity method (preparation column:ChiralPak AD, 150×4.6mm I.D., 3μm; mobile phase: A-phase $CO_2$/B-phase isopropanol (0.05% DEA), gradient: B 40%; flow rate: 2.5 ml/min; column pressure: 100 bar; column temperature 35 °C, wavelength 220 nm).

Example 15: Evaluation of compounds

The properties of the compound of the present invention were verified by activity test of compounds, integrase inhibition activity test, oil-water partition coefficient of compounds, PK test of compounds (mouse) and PK test (Crab-eating Macaque) of compounds.

The structure of the control drug involved in the above tests was as follow:

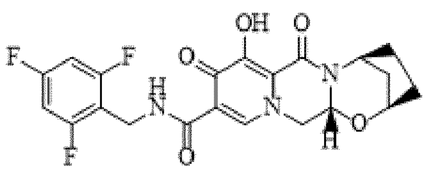

BIC

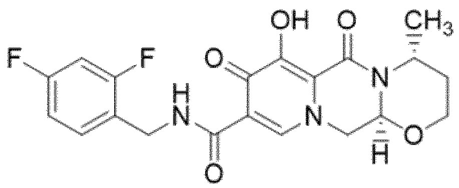

DTG

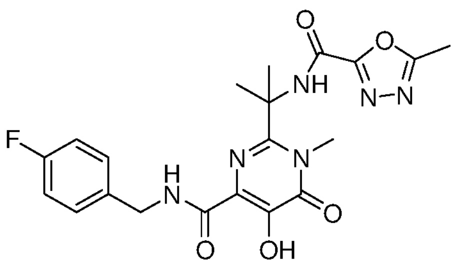

RAL

1. Activity test

1. Experimental materials

- Virus: The HIV-1 pseudovirus used in this experiment was a replication deficient virus, which is constructed by WuXi AppTec.
- HEK 293T cell line was purchased from ATCC, and Item No. is CRL-11268. Cells were cultured in DMEM medium with 10% fetal bovine serum and 1% doubleantibody.
- Reagents

| Reagent name | Supplier | Item No. |
|---|---|---|
| Fetal bovine serum (FBS) | Excellent Bio | FSP500 |
| Doubleantibody solution (PS) | Hyclone | SV30010 |
| DMEM | Gibco | 11960-051 |
| Luciferase reporter gene assay reagent Britellite plus kit | PerkinElmer | 6066769 |
| Cell viability assay reagent kit Cell Tier Glo | Promega | G7553 |

- Main instruments

| Instrument name | Supplier/Model | model |
|---|---|---|
| Multi label microplate detector Envision | PerkinElmer | Envision2104 |
| Enzyme-linked immunosorbent assay reader | Bio Tek | Synergy |

2. Experimental methods

Method for HIV-1 pseudovirus infection test

| Virus strain | cell | treatment time (days)/test | Control compound |
|---|---|---|---|
| HIV-1 pseudovirus | HEK 293T | 3/Luc | RAL |

(1) Cell plating:

On the first day, HEK 293T cells were inoculated into a 96 well test plate at the density of 55000 cells per well and 100μ per well, and incubated overnight in a 5% $CO_2$ and 37 °C incubator.

(2) Compound treatment and viral infection:

On the second day, the tested compounds (compounds provided in the example of the present invention) and the control compounds were diluted 4-fold with cell culture medium for a total of 8 concentration points, and 50μL diluted compounds were added to each well, and each well was performed duplicate.

Subsequently, the HIV-1 pseudovirus was diluted to 100 TCID50/50 μL with cell culture medium, and 50μL diluted virus was added to each well.

Cell Control (cells, no compound treatment or virus infection) and virus control (cells infected with virus, no compound treatment) were set up. The final initial test concentration of the tested compounds and control compounds was 1000 nM (Table 1), and the amount of virus infection was 100 TCID50 per well. The total volume of cell culture medium was 200μL per well, and the final concentration of DMSO in the culture medium was 0.5%.

The cells were cultured in a 5% $CO_2$ and 37 °C incubator for additional 3 days.

(3) Luciferase activity assay:

On the fifth day, in the antiviral activity test plates, 100μL cell supernatant was sucked away from each well, 100μL 2× luciferase reporter gene assay reagent Britellite plus was added in dark. The luciferase activity in each well of the test plates was detected by Envision. The data was used for sample antiviral activity analysis.

(4) Cytotoxicity test:

On the fifth day, in the cytotoxicity test plates, 100μL cell supernatant were sucked from each well, 100μL cell viability assay reagent Cell Tier Gloin was added in dark. Cell viability in each well was detected by BioTek enzyme-linked immunosorbent assay reader, and the data was used for sample cytotoxicity analysis.

(5) Data processing:

Dose-responsecurve was drawn through GraphPad Prism software. The calculation equations for antiviral activity (% Inhibition) and cell viability (% cell viability) were as follow:

Antiviral activity (%)=(reading value of test well - average of virus control)/(average of cell contol - average of virus control) × 100;

Cell viability (%)=(reading value of test well - average of medium control)/(average of cell contol - average of medium control) × 100;

The values of$EC_{50}$ and $CC_{50}$ were analyzed by GraphPad Prism (version 5) software,and nonlinear fitting analysis was used for inhibitory activity and cell viability of the compounds, and the fitting method was "log (inhibitor) vs. response -- Variable slope".

3. The HIV-1 pseudovirus experiment was applied to determinate the *in vitro* antiviral activity and cytotoxicity of the tested compound on HEK 293T cells, and the experimental results were shown in the table below.

Table 1. Results of antiviral activity of compounds

| Compound number | $EC_{50}$ (nM) | $CC_{50}$ (nM) |
|---|---|---|
| 1 | 1.08 | >1000 |
| 2 | 1.19 | >1000 |
| 3 | 5.63 | >1000 |
| 4 | 5.83 | >1000 |
| 5 | 4.45 | >1000 |
| 6 | 3.03 | >1000 |
| 7 | 11.64 | >1000 |
| 8 | 13.70 | >1000 |
| 9 | 5.09 | >1000 |
| 10 | 2.7 | >1000 |
| 11 | 2.63 | >1000 |
| 12 | 4.93 | >1000 |
| 13 | 1.05 | >1000 |
| 14 | 8.72 | >1000 |
| 15 | 13.96 | >1000 |
| 16 | 1.11 | >1000 |
| 17 | 3.73 | >1000 |
| 18 | 3.34 | >1000 |
| RAL | 3.42 | >1000 |

(continued)

| Compound number | $EC_{50}$ (nM) | $CC_{50}$ (nM) |
|---|---|---|
| DTG | 1.49 | >1000 |
| Note: $EC_{50}$: The compound concentration that protects 50% HIV-infected MT-4 cells from cell pathological changes; $CC_{50}$: The concentration at which the target compound causes pathological changes in 50% HIV-uninfected cells. | | |

The results showed that the control compounds RAL, DTG and the tested compound have antiviral activity against HIV-1 pseudo virus, the compound did not show significant cytotoxicity on HEK 293T cells, and the $CC_{50}$ value was higher than the highest test concentration.

2. Integrase activity test

A non-radioactive assay method, an integrase assay kit, was used to measure integrase activity quantitatively. HIV-1 integrase cleaves two base pairs at the 3'end of donor double stranded DNA (DS DNA) and catalyzes the integration of the cleaved DS NDA into the double stranded target DNA (TS DNA) containing a modification at 3' end, HRP labeled antibodies specifically modify against the 3'end modification, and absorbance values are read at 450 nm to quantify integrase activity.

1. Reagents and consumables:

HIV-1 Integrate Assay Kit # EZ-1700
14.5 M β- Mercaptoethanol (BME), metal bath, constant temperature incubator, 96 well culture plate (NEST)
Desktop enzyme-linked immunosorbent assay reader (SpectraMax® ID5), DMSO;
Testedcompound: Compound 1, Dolutegravir (DTG), Bictegravir (BIC)

2. Test steps

(1) Reagent preparation:

Each component was prepared according to the instructions of the kit; preparation of test sample, 5 mM DMSO solution was prepared for backup, working solution was prepared according to the initial final concentration determinated at the time of method establishment, with a 4-fold gradientdilution and 10 concentrations.

(2) Methodologyestablishment: EZ-1700 kit was used for methodology establishment;

① Azide was used for data reproducibility experiments, and antibody incubation time was determinated;
(2) Exploration of the test concentration of compound 1, DTG and BIC was carried out;

(3) Sample test

①The working solution of sample was prepared according to the initial concentration determinated during the methodology establishment process;
②Reagents were prepared according to the methods provided in the kit;
③ DS DNA coating- blocking -addition of integrase solution -addition of sample-addition of TS DNA - addition of HRP antibody -addition of TMB peroxidase substrate - TMB termination reaction -detection of light absorption value at 450 nm.

The software Graphpad Prism 8.0 was applied and the calculation equation of Log (inhibitor) vs. response variable slope was used to fit the $IC_{50}$ curve.

The $IC_{50}$ values determinated in the above tests of the compounds of the present invention are shown in the table below.

Table 2. Results of Integrase Inhibition Activity of Compounds

| Sample | $IC_{50}$ (nM) |
|---|---|
| Compound 1 | 9.19 |
| DTG | 13.60 |
| BIC | 21.24 |

Conclusion: The compounds of the present invention can inhibit the activity of HIV integrase, and have a better inhibitory effect on integrase activity compared with Dotiravir (DTG) and Bitiravir (BIC).
3.Oil-water partition coefficientof compounds
Oil-water partition coefficient of compounds

a) To a 5mL glass bottle was added 1 mL n-octanol and 1 mL pH 7.4 phosphate buffer , respectively.
b) The compound was added to a DMSO solution to prepare a compound stock solution with a concentration of 10 mM, and then 20 μL compound stock solution was added to the mixture solution of n-octanol and phosphate buffer.
c) Shaken at room temperature for 1 hourat a rate of 300/minute.
d) Kept for 30 minutes, and after the solution was completely layered, the upper and lower layers of the solution were taken separately to a 96 well deep well plate, and LC-MS/MS was used for detection via injection.
e) The aliquots of 1-octanol and aqueous solution were diluted consecutively with DMSO to obtain the final sample for LC-MS/MS analysis. Three consecutive dilutions were carried on every1-octanol phase with covering a concentration range of 2500 fold. Every final aqueous phase was diluted consecutively twice with covering a concentration range of 100 fold. The MS peak areas of these solutions were used to generate log (peak area) based on the log (relative concentration) calibration line.

$$\text{LogD (pH7.4)}=\text{LOG (Coct/Caq)}$$

Coct=sample concentration in n-octanol
Caq= sample concentration in pH 7.4 phosphate buffer

Table 3. Oil-water partition coefficient of compounds

| Sample | Log D (pH 7.4) |
|---|---|
| Control compound | Testosterone, 2.85 |
| Compound 1 | 2.2 |
| Compound 2 | 2.5 |
| Compound 6 | 2.0 |
| Compound 10 | 1.9 |
| Compound 13 | 1.5 |
| Compound 16 | 1.8 |
| DTG | 1.6 |
| Note: 1<LogD7.4<3A, relatively ideal range, as there was a good balance between dissolution and passive diffusion permeation, and these compounds usually have good intestinal absorption; 3<LogD7.4<5, due to poor solubility, these compounds have good permeability but low absorption; LogD7.4>5, low solubility results in poor absorption and bioavailability of compounds within this range. | |

Conclusion: The log D values of DTG, compound 6, compound 1 and compound 2 were 1.6, 2.0, 2.2and 2.5 at pH 7.4, respectively, indicating that these compounds have good intestinal absorption, while during the intestinal absorption, the higher of the log D value means the better lipid solubility and cell permeability. Therefore, the cell permeability of the above compounds was ranked from high to low as compound 2>compound 1>compound 6>DTG.

4. PK test of compounds(mouse)

1. Summary

ICR mice were used as test animals, the LC-MSMS was used to determinate the drug concentrations at different time in the plasma of mice,who had been administered with compounds (compound 1, compound 2, compound 6, compound 10) and positive control drug (DTG) via gavage (PO) and intravenous injection (IV). The pharmacokinetic behaviors of compounds in mice were studied and their pharmacokinetic characteristics were evaluted.

2. Experimental drug

Compound 1, Compound 2, Compound 6, Compound 10 and positive control drug (DTG).

3. Experimental animals

SPF grade ICR male mice, 10 weeks old, about 32g weight, 6 mice in each group, 48 mice in total.

4. Experimental formulation

Gavage administration group (PO): 5% DMSO/10% Solutol/85% (20% Captisol in water);
Intravenous injection group (IV): 5% DMSO/10% Solutol/85% (20% Captisol in water).

5. Administration

The mice were not fed until 5pm on the day before administration, and fasted overnight with water. The mice in Group IV were administered with 2 mg/kg, while those in Group PO were administered with 10 mg/kg. Feeding was resumed 2 hours after administration.

6. Sampling

For mice administered by gavage, 20μL blood was collected from the saphenous vein in calf before administration and 0.25, 0.5, 1, 2, 4, 8, and 24 hours after administration, respectively, and placed in EDTA-K2 anticoagulant blood collection tubes, and then the blood would be placed on ice until centrifuged to obtain plasma samples. The plasma samples were centrifuged at 4 °C (3500 rpm, 10 min), and after separation, the samples were transferred to EP tubes and stored at -80 °C for detection.

For mice admistrated by intravenous injection, 20μL blood was collected from the saphenous vein in calf before administration and 0.0833, 0.25, 0.5, 1, 2, 4, 8and 24 hours after administration, respectively, and placed in EDTA-K2 anticoagulant blood collection tubes, and then the blood would be placed on ice until centrifuged to obtain a plasma sample. The plasma sampleswerecentrifuged at 4 °C (3500 rpm, 10 min), and after separation, the sampleswere transferred to EP tubes and stored at -80 °C for detection.

7. Detection

The level of tested compounds in blood plasma of mice, who had orally administrated with different concentrations of drugs were determinated: 10μL mouse blood at each time point after administration was taken, 100 μL methanol containing an internal standard (100 ng/mL) was added, vortex mixed for 1 minute, centrifuged at 4 °C (12000 rmp, 5 minutes), and 1 μL supernatant was taken from the plasma samples for LC-MS/MS analysis.

8. Pharmacokinetic parameter results

Table 4. PK results of compounds in mice

| Parameter | | DTG | Compound 1 | Compound 2 | Compound 6 | Compound 10 |
|---|---|---|---|---|---|---|
| PO (10mg/kg) | $T_{1/2}$ (hr) | 3.70 ± 0.09 | 4.37 ± 0.47 | 1.94 ± 0.04 | 2.68 ± 0.66 | 1.53 ± 1.06 |
| | Bioavailability | 46% | 57% | 50.8% | 42.5% | 32.9% |

9. Summary

The results of PK test in mice showed that compound 1, 2, 6 and 10 had good bioavailability. Wherein, the bioavailability and half-life data of compound 1 were superior to the positive control drug DTG.

5. PK test of compounds (crab-eating macaque)

1. Summary

Crab-eating macaques were used as test animal, the LC-MS/MS was used to determinate the drug concentration at different times in the plasma of crab-eating macaques, who had been administered with compound 1 via gavage (PO). The pharmacokinetic behaviors of compounds of present disclosure in crab-eating macaques were studied and their pharmacokinetic characteristics were evaluated.

2. Experimental drug

Compound 1.

3. Experimental animals

Crab-eating macaques(*Macaca fascicularis*), common grade, male, approximately 2.5-4 years old, 2-5 kg weight, 3 in each group.

4. Experimental formulation

Compound 1 and a solid substrate (hydroxypropyl methylcellulose acetate succinate) are dissolved in an organic solvent, and the solvent was removed by heating evaporation, or removed by a spray dryer to obtain compound 1 loaded on the solid substrate.

Compound 1 loaded on the solid substrate, which was obtained according to the above steps,was slowly added to a suitable container with approximately 80% final volume of vehicle while stirring. Stirred until it is visually uniform, and if necessary, the formulation shall be subjected to ultrasonic treatment. Vehicle was added to give the desired concentration. The obtained formulation shall be consecutively stirred until a visually uniform formulation was obtained, and if necessary, the formulation shall be subjected to ultrasonic treatment. The administration concentration with 20 mg/mL API concentrationwas prepared, and the preparation method for above vehicle was: approximately 30% total volume of purified water was added to an suitable container, and heated up to approximately 80 °C-90 °C, the required volume of Tween 80 was added slowly to the container, while stirred until visually uniform. Purified water was added to the final volume. Stirred until a visually uniform solution was obtained.

5. Administration

Crab-eating macaques were fast overnight and given free acess to water. The macaques in PO group were administered with 100 mg/kg. Feeding was resumed 2 hours after administration.

6. Sampling

For crab-eating macaques administered by gavage, 20μL blood were collected from the veins in hind limb before administration and 0.25, 0.5, 1, 2, 4, 8, 24, 48, 72 and 96 hours after administration, respectively,and placed in EDTA-K2 anticoagulant blood collection tubes, and then the blood would be placed on ice until centrifuged to obtain plasma samples. The plasma samples were centrifuged at 4 °C (3500 rpm, 10 min), and after separation, the plasma samples were transferred to EP tubes and stored at -80 °C for detection.

7. Detection

The level of tested compounds in the plasma of crab eating macaques, who had been orally administrated with different concentrations of drugwere determinated: 10μL macaque blood at each time point after administration was taken, 100 μL methanol containing an internal standard (100 ng/mL) was added, vortex mixed for 1 minute, centrifuged at 4 °C (12000 rmp, 5 minutes), and 1μL supernatant was taken from the plasma samples for LC-MS/MS analysis.

8. Pharmacokinetic parameter results

Table 5. PK results of compounds in crab eating macaques

| PO Time (hr) | PO 100mg/kg | PO 30mg/kg |
|---|---|---|
| | Mean ± SD | Mean ± SD |
| $T_{1/2}$ (hr) | 19.8 ± 4.78 | 15.7 ± 5.49 |
| $T_{max}$ (hr) | 18.67 ± 25.40 | 4.00 ± 0.00 |
| $C_{max}$ (ng/mL) | 4037 ± 871 | 1797 ± 534 |
| $AUC_{0-t}$ (ng·hr/mL) | 140009 ± 44187 | 22880 ± 3814 |
| $AUC_{0-\infty}$ (ng·hr/mL) | 163847 | 23130 ± 3838 |
| MRT (hr) | 26.85 | 14.1 ± 2.68 |

9. Conclusion

The results of this test showed that the $T_{1/2}$ of oral administration of compound 1 (30, 100 mg/kg) in crab-eating macaques were 15.7 ± 5.49 h and 19.8 ± 4.78 h. The preferably compound 1 of the present invention had a long half-life, which can reduce the frequency of medication for AIDS patients, overcome the inconvenience of multiple administrations, and more meet the requirements of medical administration.

The above are only preferably examples of the present invention and is not intended to limit the present invention, and the present invention may have various modifications and variations for those skilled in the art. Any modifications, equivalent substitutions, improvements, etc. made within the spirit and principles of the present invention shall be included within the scope of protection of the present invention.

## Claims

1. A polycyclic carbamoyl pyridone derivative, comprising a compound shown in formula (I) -1 or formula (I) -2, an isomer, a pharmaceutically acceptable salt, a hydrate, or a solvate thereof,

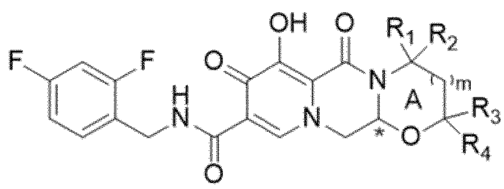

Formula (I) -1;

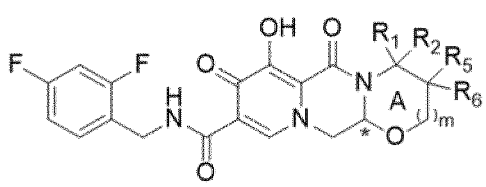

Formula (I) -2,

wherein $R_1$, $R_2$, $R_3$, $R_4$, $R_5$ and $R_6$ are each independently selected from the group consisting of hydrogen, halogen, substituted or unsubstituted alkyl, substituted or unsubstituted cycloalkyl, substituted or unsubstituted alkenyl, substituted or unsubstituted alkynyl, $-OR_7$, substituted or unsubstituted aryl, substituted or unsubstituted monocyclic heteroaryl, substituted or unsubstituted monocyclic heterocyclyl, and cyano;
and at least one group of functional groups in (1) - (3) below together with the carbon atom to which ring A connected forms a spirolcycle or spiroheterocycle:

(1) $R_1$ and $R_2$; (2) $R_3$ and $R_4$; (3) $R_5$ and $R_6$;
$R_7$ is selected from the group consisting of hydrogen, substituted or unsubstituted alkyl, substituted or unsubstituted alkenyl, and substituted or unsubstituted alkynyl;
M is any natural number between 0 and 5.

**2.** The polycyclic carbamoyl pyridone derivative according to claim 1, wherein $R_1$, $R_2$, $R_3$, $R_4$, $R_5$ and $R_6$ are each independently selected from any one of the group consisting of hydrogen, halogen, C1-4 alkyl, halogenated C1-C4 alkyl, C2-C6 alkenyl, C2-C6 alkynyl, $-OR_7$, C3-C8 cycloalkyl, C6-C10 aryl, 5-6-membered monocyclic heteroaryl, 4-6 membered saturated monocyclic heterocyclyl, and cyano;

and at least one group of functional groups in (1) - (3) below together with the carbon atoms to which ring A connected forms a 3-8-membered spirolcycle or a 3-8-membered spiroheterocycle: (1) $R_1$ and $R_2$; (2) $R_3$ and $R_4$; (3) $R_5$ and $R_6$;
$R_7$ is selected from the group consisting of hydrogen, C1-4 alkyl, C2-C6 alkenyl, C2-C6 alkynyl, and halogenated C1-C4 alkyl;
m is 0, 1, 2 or 3.

**3.** The polycyclic carbamoyl pyridone derivative according to claim 1 or 2, wherein, the tertiary carbon atom to which ring A and the fused pyrazine ring connected in the compound shown in formula (I) -1 and formula (I) -2 is chiral;

the compound shown in Formulas (I) -1 and (I) -2 include a single configuration compound or a mixture of isomers; the isomers include at least one of tautomers, cis-trans isomers, mesoisomers, and optical isomers with enantiomeric or non enantiomeric relationships.

**4.** The polycyclic carbamoyl pyridone derivative according to claim 1, wherein, the polycyclic carbamoyl pyridone derivative is selected from any one of the group consisting of the compounds shown in the following structural formulas:

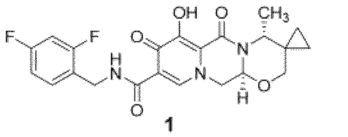
1

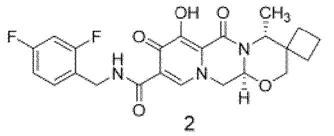
2

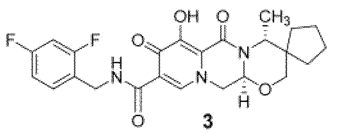
3

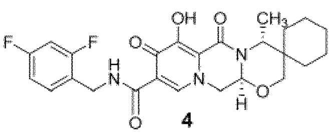
4

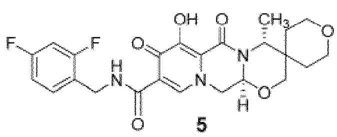
5

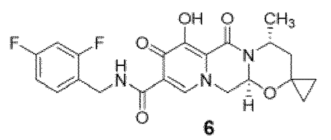
6

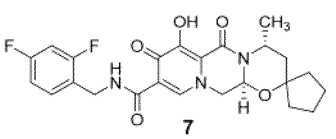
7

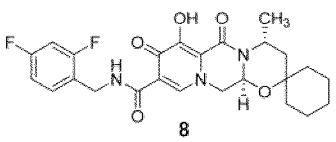
8

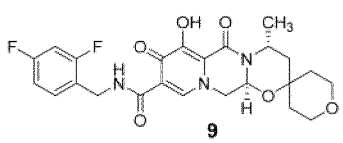
9

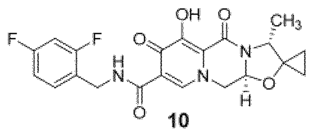
10

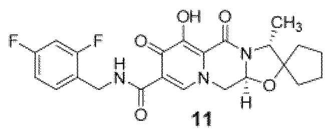
11

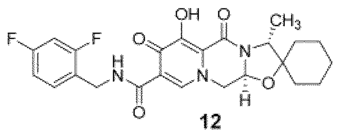
12

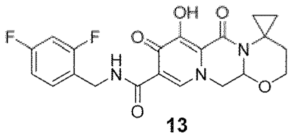
13

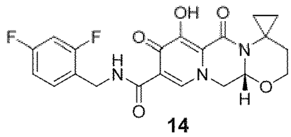
14

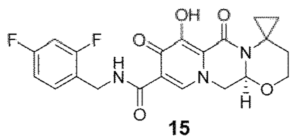
15

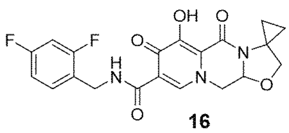
16

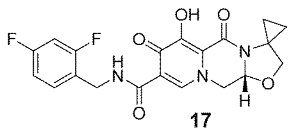
17

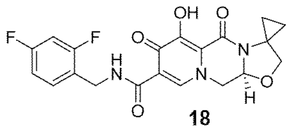
18

.

5. The polycyclic carbamoyl pyridone derivative according to claim 1, wherein, the polycyclic carbamoyl pyridone derivative is a compound having the following structural formula;

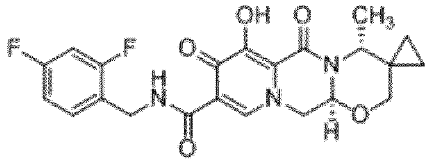

.

6. A preparation method for the polycyclic carbamoyl pyridone derivative according to any one of claims 1-5, wherein, the polycyclic carbamoyl pyridone derivative is synthesized according to the following synthesis route:

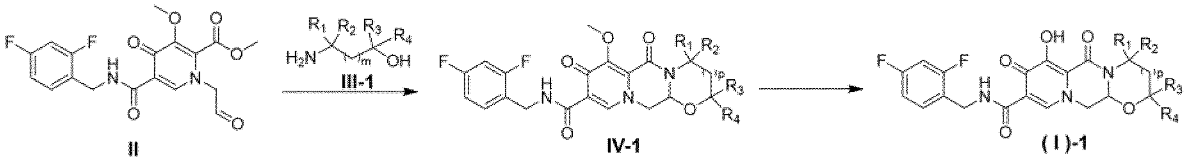

II IV-1 (I)-1

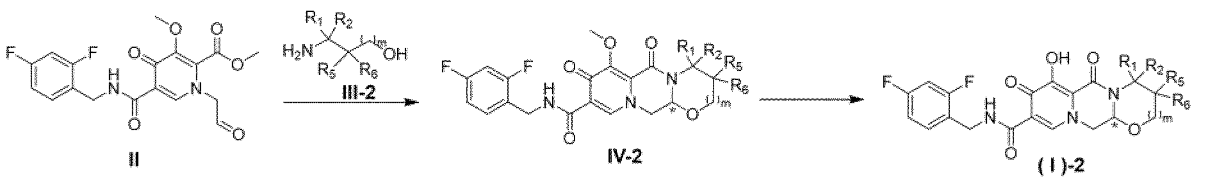

II IV-2 (I)-2

7. The preparation method according to claim 6, wherein, the conditions for preparing intermediate IV-1 include: the molar ratio between compound II and compound III-1 is 1:1-1:5, the temperature is 30-100 °C; the time is 5 minutes -16 hours;

the conditions for preparing intermediate IV-2 include: the molar ratio between compound II and compound III-2 is 1:1-1:5, the temperature is 30-100 °C; the time is 5 minutes -16 hours;
the conditions for preparing the compound shown in formula (I) -1 include: the temperature is 30-100 °C; the time is 5 minutes - 16 hours;
the conditions for preparing the compound shown in formula (I) -2 include: the temperature is 30-100 °C; the time is 5 minutes -16 hours.

8. A pharmaceutical composition, comprising a polycyclic carbamoyl pyridone derivative according to any one of claims 1-5, an isomer, a pharmaceutically acceptable salt, a hydrate, or a solvate thereof, and pharmaceutically acceptable carriers.

9. The pharmaceutical composition according to claim 8, wherein, the pharmaceutically acceptable carriers are selected from the group consisting of injection water, freeze-dried powder excipients, and oral preparation excipients.

**10.** A use of a polycyclic carbamoyl pyridone derivative according to any one of claims 1-5 or a pharmaceutical composition according to claims 8 or 9 in the preparation of drugs for preventing and/or treating diseases mediated by HIV infection.

**INTERNATIONAL SEARCH REPORT**

International application No.
**PCT/CN2022/130550**

**A. CLASSIFICATION OF SUBJECT MATTER**

C07D 498/20(2006.01)i;  A61K 31/537(2006.01)i;  A61P 31/18(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

WPI, EPODOC, CNPAT, STN(结构检索): 艾滋病, HIV, AIDS

**C. DOCUMENTS CONSIDERED TO BE RELEVANT**

| Category* | Citation of document, with indication, where appropriate, of the relevant passages | Relevant to claim No. |
|---|---|---|
| PX | CN 114230579 A (NANJING AIDI MEDICAL SCIENCE AND TECHNOLOGY LTD., CO. et al.) 25 March 2022 (2022-03-25) claims 1-10 | 1-10 |
| X | CN 112521386 A (ZHOU YUTIAN et al.) 19 March 2021 (2021-03-19) claims 3, 5 and 6-9, and description, paragraph [0259] | 1-10 |
| X | WO 2006116764 A1 (SHIONOGI & CO., LTD. et al.) 02 November 2006 (2006-11-02) claims 12 and 51-54, description, page 65 paragraph [0016], and embodiments C-1 | 1-10 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 December 2022** | **19 December 2022** |

| Name and mailing address of the ISA/CN | Authorized officer |
|---|---|
| **China National Intellectual Property Administration (ISA/ CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/130550**

| Patent document cited in search report | Publication date (day/month/year) | Patent family member(s) | Publication date (day/month/year) |
|---|---|---|---|
| CN 114230579 A | 25 March 2022 | None | |
| CN 112521386 A | 19 March 2021 | None | |
| WO 2006116764 A1 | 02 November 2006 | US 2019152990 A1 | 23 May 2019 |
| | | US 2017369509 A1 | 28 December 2017 |
| | | NO 2021018 I1 | 10 May 2021 |
| | | KR 20140097438 A | 06 August 2014 |
| | | SI 3284520 T1 | 30 October 2019 |
| | | US 2017145033 A1 | 25 May 2017 |
| | | PL 3045206 T3 | 31 August 2018 |
| | | DK 3045206 T3 | 22 May 2018 |
| | | CY 1115151 T1 | 09 December 2015 |
| | | PL 2465580 T3 | 30 May 2014 |
| | | PT 3372281 T | 14 October 2021 |
| | | DK 2465580 T3 | 10 March 2014 |
| | | PT 3284520 T | 10 September 2019 |
| | | EP 3045206 A1 | 20 July 2016 |
| | | NL 301109 I1 | 15 September 2021 |
| | | HK 1107227 A1 | 03 April 2008 |
| | | US 2012115875 A1 | 10 May 2012 |
| | | IL 225207 A | 30 November 2017 |
| | | KR 20130133061 A | 05 December 2013 |
| | | LT 3284520 T | 25 September 2019 |
| | | LU 92446 I2 | 29 October 2015 |
| | | ES 2667868 T3 | 14 May 2018 |
| | | US 2017253616 A1 | 07 September 2017 |
| | | FI 3187225 T3 | 28 February 2022 |
| | | CA 2606282 A1 | 02 November 2006 |
| | | HU S2100022 I1 | 28 July 2021 |
| | | HK 1172282 A1 | 19 April 2013 |
| | | ES 2892304 T3 | 03 February 2022 |
| | | HU E037795 T2 | 28 September 2018 |
| | | KR 20080009733 A | 29 January 2008 |
| | | HU E044978 T2 | 28 November 2019 |
| | | SI 3187225 T1 | 29 April 2022 |
| | | US 2020339598 A1 | 29 October 2020 |
| | | NL 300676 I2 | 11 October 2016 |
| | | US 2015232479 A1 | 20 August 2015 |
| | | US 2014200209 A1 | 17 July 2014 |
| | | US 2019284208 A1 | 19 September 2019 |
| | | ES 2906792 T3 | 20 April 2022 |
| | | EP 2527007 A1 | 28 November 2012 |
| | | ES 2446324 T3 | 07 March 2014 |
| | | EA 200702080 A1 | 28 April 2008 |
| | | EP 3284519 A1 | 21 February 2018 |
| | | EP 3187225 A1 | 05 July 2017 |
| | | HK 1251191 A1 | 25 January 2019 |
| | | LT 3045206 T | 11 June 2018 |
| | | US 2016137666 A1 | 19 May 2016 |
| | | IL 215788 D0 | 29 December 2011 |
| | | ES 2743531 T3 | 19 February 2020 |

Form PCT/ISA/210 (patent family annex) (January 2015)

**INTERNATIONAL SEARCH REPORT**
**Information on patent family members**

International application No.
**PCT/CN2022/130550**

| Patent document cited in search report | Publication date (day/month/year) | Patent family member(s) | Publication date (day/month/year) |
|---|---|---|---|
| | | NZ 562339 A | 28 January 2011 |
| | | US 2013172559 A1 | 04 July 2013 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Journal of Medical Chemistry,* 2016, vol. 59 (8), 3732-3749